# EUROPEAN PATENT APPLICATION

(11) **EP 4 140 496 A1**
(43) Date of publication of application: **01.03.2023**
(21) Application number: 21793803.4
(22) Date of filing: 20.04.2021
(51) Int. Cl.: A61K 38/17, A61K 38/26, A61K 38/22, A61K 47/68, A61P 3/06, C07K 14/605, C07K 14/575, C07K 14/72

(54) **PREVENTIVE OR THERAPEUTIC PHARMACEUTICAL COMPOSITION FOR HYPERLIPIDEMIA COMPRISING TRIPLE AGONIST ACTING ON ALL OF GLUCAGON, GLP-1 AND GIP RECEPTORS, OR CONJUGATE THEREOF, AND PREVENTIVE OR THERAPEUTIC METHOD**

(30) Priority: 20.04.2020 KR 20200047733
(71) Applicant: Hanmi Pharm. Co., Ltd., Hwaseong-si, Gyeonggi-do 18536 (KR)
(72) Inventor: JO, Hyo Sang, Hwaseong-si, Gyeonggi-do 18469 (KR); KIM, Jung Kuk, Hwaseong-si, Gyeonggi-do 18469 (KR); LEE, A Ram, Hwaseong-si, Gyeonggi-do 18469 (KR); KIM, Sang Yun, Hwaseong-si, Gyeonggi-do 18469 (KR)
(74) Representative: Mewburn Ellis LLP
(86) International application number: PCT/KR2021/004961
(87) International publication number: WO 2021/215801

(57) **Abstract**

Provided is a use of a triple glucagon/GLP-1/GIP receptor agonist or a long-acting conjugate thereof for treatment of hyperlipidemia.

## Description

### [Technical Field]

The present invention relates a use of a trigonal glucagon/GLP-1/GIP receptor agonist or a conjugate thereof for treatment of hyperlipidemia.

### [Background Art]

Lipids include cholesterol and triglycerides, and cholesterol is classified into low-density lipoprotein (LDL) cholesterol and high-density lipoprotein (HDL) cholesterol. In this regard, unlike dyslipidemia, in which levels of cholesterol and triglycerides in blood are outside of normal ranges (including both increases and decreases), and hypercholesterolemia, in which a total cholesterol level in blood is high, hyperlipidemia is a general term for diseases in which one of the LDL cholesterol level and the triglyceride level or both are high in blood and distinguished from dyslipidemia and hypercholesterolemia. Accordingly, since pathogenesis and patients' conditions for these diseases are different from each other, different methods for treatment and diet control are required. For example, in the case of dyslipidemia caused by abnormally regulated lipid levels in the body, an abnormally low cholesterol level may be a problem, and it is important to increase the level of cholesterol, which is essential for vital activity via diets such as intake of health supplements, in a manner different from that for hyperlipidemia.

When the levels of LDL cholesterol and/or triglycerides are abnormally high in blood, they may accumulate on walls of blood vessels causing inflammation, leading to cardiovascular disease, cerebrovascular disease, and peripheral arterial disease. Among these, cardiovascular disease and cerebrovascular disease along with cancer are the three major causes of death in Korea. Hyperlipidemia has increased with the rise in obesity due to Western-style eating habits, and the number of patients with hyperlipidemia is increasing by 10% every year in Korea. Obesity, drinking alcohol, and the like have been known as causes of hyperlipidemia. Among these, obesity is the largest cause.

Various efforts have been made to adjust the blood lipid level for prevention and/or treatment of hyperlipidemia. For example, statins, which have inhibitory effects on cholesterol synthesis by acting as an HMG-CoA reductase (HMGCR) inhibitor, ezetimibe, which has inhibitory effects on reabsorption of cholesterol by inhibiting Niemann-Pick C1-Like 1 (NPC1 L1) in the small intestine, and evolocumab, which inhibits proprotein convertase subtilisinlkexin type 9 (PCSK9) that inhibits absorption of LDL, have been developed and used as therapeutic agents (US Endocrinology, 2011;7(1):23-9, Am J Pharm Benefits. 2010;2(4):267-274, N Engl J Med 2014; 370:1809-1819).

However, treatment with statin-based drugs is disadvantageous in that cholesterol levels are not sufficiently adjusted thereby in many cases, and high-dosage or long-term use may cause hepatotoxicity and myopathic disorders. Use of ezetimibe is disadvantageous in that effects thereof on adjusting the blood lipid level are significantly lower than those of conventional drugs in clinical practices. Evolocumab has been reported to be ineffective in patients with genetic mutation in an LDL receptor (LDLR). As described above, since currently commercialized drugs act only on a single target, they are not effective in removing lipids from the blood of patients with genetic mutation or patients with a super high risk of hyperlipidemia. Therefore, there is a need to develop drugs acting on multiple targets and exhibiting efficacy in a wide range of hyperlipidemia patients.

Meanwhile, glucagon-like peptide-1 (GLP-1) and glucose-dependent insulinotropic polypeptide (GIP), as representative gastrointestinal hormones and neurological hormones, are substances involved in regulation of blood glucose concentration in accordance with food intake. Glucagon, as a peptide hormone secreted by the pancreas, is also involved in regulation of blood glucose concentration and lipid metabolism together with the two substances.

Specifically, GLP-1, as a hormone secreted by the small intestine when stimulated by food intake, promotes secretion of insulin by the pancreas in a blood glucose concentration-dependent manner and assists in lowering the blood glucose concentration. In addition, the GLP-1 acts as a satiety factor to reduce food intake by slowing digestion of the stomach and delaying time for gastric emptying. Furthermore, effects on reducing food intake and losing body weight have been reported when administered to rats, and these effects are confirmed to appear identically in both normal and obese cases, thereby indicating potential as a therapeutic agent for obesity.

Together with the GLP-1, GIP, as a gastrointestinal hormone secreted upon stimulation of food intake, consists of 42 amino acids and is secreted from K cells of the small intestine. Effects of GIP on increasing activity of GLP-1 and inhibiting inflammation have been reported by inhibiting Apolipoprotein B 48 (ApoB48) in enterocytes, resulting in reduction in release of lipids absorbed by the small intestine into capillaries.

Glucagon is produced by the pancreas when blood glucose levels drop as a result of other medications or diseases, or deficiency in hormones or enzymes. Glucagon sends a signal for glycogen breakdown in the liver and a subsequent glucose release to have a role in increasing blood glucose levels to a normal range. In addition to the effect of increasing the blood glucose levels, effects of glucagon on suppressing appetite in animals and humans and promoting browning adipocytes and energy expenditure have been reported.

### [Disclosure]

### [Technical Problem]

An object of the present invention is to provide a pharmaceutical composition for preventing or treating hyperlipidemia including a peptide with activity to a glucagon receptor, a glucagon-like peptide-1 (GLP-1) receptor, and a glucose-dependent insulinotropic polypeptide (GIP) receptor, or a conjugate thereof.

Another object of the present invention is to provide a method of preventing or treating hyperlipidemia including administering the peptide, the conjugate thereof, or the composition including the same to an individual in need thereof.

Another object of the present invention is to provide a use of the peptide, the conjugate thereof, or the composition including the same for preparation of medicaments for preventing or treating hyperlipidemia.

### [Technical Solution]

An aspect of the present invention provides a pharmaceutical composition for preventing or treating hyperlipidemia including a peptide with activity to a glucagon receptor, a glucagon-like peptide-1 (GLP-1) receptor, and a glucose-dependent insulinotropic polypeptide (GIP) receptor.

In a specific embodiment, the peptide is characterized in that an amino acid sequence added thereto is derived from an amino acid sequence of native GLP-1, native GIP, or native exendin-4.

In another specific embodiment, it is characterized in that the peptide is a peptide including an amino acid sequence represented by General Formula 1 below:

Xaa1-Xaa2-Xaa3-Gly-Thr-Phe-Xaa7-Ser-Asp-Xaa10-Ser-Xaa12-Xaa13 -Xaa 14-Xaa 15-Xaa 16-Xaa 17-Xaa 18-Xaa 19-Xaa20-Xaa21 -Phe-Xaa23-Xaa24-Trp-Leu-Xaa27-Xaa28-Xaa29-Xaa30-R1 (General Formula 1, SEQ ID NO: 103)

In General Formula 1 above, the peptide is characterized in that Xaa1 is histidine (His, H), 4-imidazoacetyl (CA), or tyrosine (Tyr, Y), Xaa2 is glycine (Gly, G), α-methyl-glutamic acid, or aminoisobutyric acid (Aib), Xaa3 is glutamic acid (Glu, E) or glutamine (Gln, Q), Xaa7 is threonine (Thr, T) or isoleucine (Ile, I), Xaa10 is leucine (Leu, L), tyrosine (Tyr, Y), lysine (Lys, K), cysteine (Cys, C), or valine (Val, V), Xaa12 is lysine (Lys, K), serine (Ser, S), or isoleucine (Ile, I), Xaa13 is glutamine (Gln, Q), tyrosine (Tyr, Y), alanine (Ala, A), or cysteine (Cys, C), Xaa14 is leucine (Leu, L), methionine (Met, M), or tyrosine (Tyr, Y), Xaa15 is cysteine (Cys, C), aspartic acid (Asp, D), glutamic acid (Glu, E), or leucine (Leu, L), Xaa16 is glycine (Gly, G), glutamic acid (Glu, E), or serine (Ser, S), Xaa17 is glutamine (Gln, Q), arginine (Arg, R), isoleucine (lie, I), glutamic acid (Glu, E), cysteine (Cys, C), or lysine (Lys, K), Xaa18 is alanine (Ala, A), glutamine (Gln, Q), arginine (Arg, R), or histidine (His, H), Xaa19 is alanine (Ala, A), glutamine (Gln, Q), cysteine (Cys, C), or valine (Val, V), Xaa20 is lysine (Lys, K), glutamine (Gln, Q), or arginine (Arg, R), Xaa21 is glutamic acid (Glu, E), glutamine (Gln, Q), leucine (Leu, L), cysteine (Cys, C), or aspartic acid (Asp, D), Xaa23 is isoleucine (Ile, I) or valine (Val, V), Xaa24 is alanine (Ala, A), glutamine (Gln, Q), cysteine (Cys, C), asparagine (Asn, N), aspartic acid (Asp, D), or glutamic acid (Glu, E), Xaa27 is valine (Val, V), leucine (Leu, L), lysine (Lys, K), or methionine (Met, M), Xaa28 is cysteine (Cys, C), lysine (Lys, K), alanine (Ala, A), asparagine (Asn, N), or aspartic acid (Asp, D), Xaa29 is cysteine (Cys, C), glycine (Gly, G), glutamine (Gln, Q), threonine (Thr, T), glutamic acid (Glu, E), or histidine (His, H), Xaa30 is cysteine (Cys, C), glycine (Gly, G), lysine (Lys, K), or histidine (His, H), or is absent, and
R1 is cysteine (Cys, C), GKKNDWKHNIT (SEQ ID NO: 106), m-SSGAPPPS-n (SEQ ID NO: 107), or m-SSGQPPPS-n (SEQ ID NO: 108), or is absent, wherein m is -Cys-, -Pro-, or -Gly-Pro-, and n is -Cys-, -Gly-, -Ser-, or -His-Gly-, or is absent.

In the composition according to any one of the specific embodiments, the peptide is characterized in that Xaa14 is leucine or methionine, and Xaa15 is cysteine, aspartic acid, or leucine in General Formula 1.

In the composition according to any one of the specific embodiments, the peptide is characterized in that, in General Formula 1 above, Xaa2 is glycine, α-methyl-glutamic acid, or Aib, Xaa7 is threonine, Xaa10 is tyrosine, cysteine, or valine, Xaa12 is lysine or isoleucine, Xaa13 is tyrosine, alanine, glutamine, or cysteine, Xaa14 is leucine, cysteine, or methionine, Xaa15 is cysteine, leucine, glutamic acid, or aspartic acid, Xaa17 is glutamine, arginine, isoleucine, cysteine, glutamic acid, or lysine, Xaa18 is alanine, glutamine, arginine, or histidine, Xaa19 is alanine, glutamine, valine, or cysteine, Xaa20 is lysine, arginine, or glutamine, Xaa21 is glutamic acid, glutamine, leucine, cysteine, or aspartic acid, Xaa23 is isoleucine or valine, Xaa24 is cysteine, alanine, glutamine, asparagine, glutamic acid, or aspartic acid, and Xaa27 is leucine or lysine.

In the composition according to any one of the specific embodiments, it is characterized in that the peptide is a peptide including an amino acid sequence of General Formula 2 below.

Xaa1-Xaa2-Gln-Gly-Thr-Phe-Thr-Ser-Asp-Xaa10-Ser-Lys-Xaa 1 3-Xaa1 4-Xaa15-Xaa16-Xaa17-Xaa18-Xaa19-Xaa20-Xaa21-Phe-Xaa23-Xaa24-Trp-L eu-Leu-Xaa28-Xaa29-Xaa30-Xaa31-Ser-Ser-Gly-Gln-Pro-Pro-Pro-Ser-Xaa40 (General Formula 2, SEQ ID NO: 104)

In General Formula 2 above, the peptide is characterized in that Xaa1 is 4-imidazoacetyl, histidine, or tyrosine, Xaa2 is glycine, α-methyl-glutamic acid, or Aib, Xaa10 is tyrosine, or cysteine, Xaa13 is alanine, glutamine, tyrosine, or cysteine, Xaa14 is leucine, methionine, or tyrosine, Xaa15 is aspartic acid, glutamic acid, or leucine, Xaa16 is glycine, glutamic acid, or serine, Xaa17 is glutamine, arginine, isoleucine, glutamic acid, cysteine, or lysine, Xaa18 is alanine, glutamine, arginine, or histidine, Xaa19 is alanine, glutamine, cysteine, or valine, Xaa20 is lysine, glutamine, or arginine, Xaa21 is cysteine, glutamic acid, glutamine, leucine, or aspartic acid, Xaa23 is isoleucine or valine, Xaa24 is cysteine, alanine, glutamine, asparagine, or glutamic acid, Xaa28 is lysine, cysteine, asparagine, or aspartic acid, Xaa29 is glycine, glutamine, cysteine, or histidine, Xaa30 is cysteine, glycine, lysine, or histidine, Xaa31 is proline or cysteine, and Xaa40 is cysteine, or is absent.

In the composition according to any one of the specific embodiments, in General Formula 1 above, the peptide is characterized in that Xaa2 is glycine, α-methyl-glutamic acid, or Aib, Xaa7 is threonine, Xaa10 is tyrosine, cysteine, or valine, Xaa12 is lysine or isoleucine, Xaa13 is tyrosine, alanine, or cysteine, Xaa14 is leucine or methionine, Xaa15 is cysteine or aspartic acid, Xaa17 is glutamine, arginine, isoleucine, cysteine, or lysine, Xaa18 is alanine, arginine, or histidine, Xaa19 is alanine, glutamine, or cysteine, Xaa20 is lysine or glutamine, Xaa21 is glutamic acid, cysteine, or aspartic acid, Xaa23 is valine, Xaa24 is alanine, glutamine, cysteine, asparagine, or aspartic acid, and Xaa27 is leucine or lysine.

In the composition according to any one of the specific embodiments, in General Formula 2 above, the peptide is characterized in that Xaa13 is alanine, tyrosine, or cysteine, Xaa15 is aspartic acid or glutamic acid, Xaa17 is glutamine, arginine, cysteine, or lysine, Xaa18 is alanine, arginine, or histidine, Xaa21 is cysteine, glutamic acid, glutamine, or aspartic acid, Xaa23 is isoleucine or valine, Xaa24 is cysteine, glutamine, or asparagine, Xaa28 is cysteine, asparagine, or aspartic acid, Xaa29 is glutamine, cysteine, or histidine, and Xaa30 is cysteine, lysine, or histidine.

In the composition according to any one of the specific embodiments, in General Formula 1 above, the peptide is characterized in that Xaa2 is α-methyl-glutamic acid or Aib, Xaa7 is threonine, Xaa10 is tyrosine or cysteine, Xaa12 is lysine or isoleucine, Xaa13 is tyrosine, alanine, or cysteine, Xaa14 is leucine or methionine, Xaa15 is cysteine or aspartic acid, Xaa16 is glutamic acid, Xaa17 is arginine, isoleucine, cysteine, or lysine, Xaa18 is alanine, arginine, or histidine, Xaa19 is alanine, glutamine, or cysteine, Xaa20 is lysine or glutamine, Xaa21 is glutamic acid or aspartic acid, Xaa23 is valine, Xaa24 is glutamine, asparagine, or aspartic acid, Xaa27 is leucine, and Xaa28 is cysteine, alanine, asparagine, or aspartic acid.

In the composition according to any one of the specific embodiments, in General Formula 1 above, the peptide is characterized in that Xaa1 is histidine or 4-imidazoacetyl, Xaa2 is α-methyl-glutamic acid or Aib, Xaa3 is glutamine, Xaa7 is threonine, Xaa10 is tyrosine, Xaa12 is isoleucine, Xaa13 is alanine or cysteine, Xaa14 is methionine, Xaa15 is aspartic acid, Xaa16 is glutamic acid, Xaa17 is isoleucine or lysine, Xaa18 is alanine or histidine, Xaa19 is glutamine or cysteine, Xaa20 is lysine, Xaa21 is aspartic acid, Xaa23 is valine, Xaa24 is asparagine, Xaa27 is leucine, Xaa28 is alanine or asparagine, Xaa29 is glutamine or threonine, and Xaa30 is cysteine or lysine, or is absent.

In the composition according to any one of the specific embodiments, it is characterized in that the peptide is a peptide including an amino acid sequence of General Formula 3 below.

Xaa1-Xaa2-Gln-Gly-Thr-Phe-Thr-Ser-Asp-Tyr-Ser-Lys-Xaa13-Leu-Asp -G I u-Xaa 17-Xaa 18-Xaa 19-Lys-Xaa21-Ph e-Va I-Xaa24-Trp-Leu-Leu-Xaa28-Xa a29-Xaa30-Xaa31-Ser-Ser-Gly-Gln-Pro-Pro-Pro-Ser-Xaa40 (General Formula 3, SEQ ID NO: 105)

In the General Formula 3, the peptide is characterized in that Xaa1 is histidine or tyrosine, Xaa2 is α-methyl-glutamic acid or Aib, Xaa13 is alanine, tyrosine or cysteine, Xaa17 is arginine, cysteine, or lysine, Xaa18 is alanine or arginine, Xaa19 is alanine or cysteine, Xaa21 is glutamic acid or aspartic acid, Xaa24 is glutamine or asparagine, Xaa28 is cysteine or aspartic acid, Xaa29 is cysteine, histidine, or glutamine, Xaa30 is cysteine or histidine, Xaa31 is proline or cysteine, and Xaa40 is cysteine, or is absent.

In the composition according to any one of the specific embodiments, the peptide is characterized in that R1 is cysteine, GKKNDWKHNIT (SEQ ID NO: 106), CSSGQPPPS (SEQ ID NO: 109), GPSSGAPPPS (SEQ ID NO: 110), GPSSGAPPPSC (SEQ ID NO: 111), PSSGAPPPS (SEQ ID NO: 112), PSSGAPPPSG (SEQ ID NO: 113), PSSGAPPPSHG (SEQ ID NO: 114), PSSGAPPPSS (SEQ ID NO: 115), PSSGQPPPS (SEQ ID NO: 116), or PSSGQPPPSC (SEQ ID NO: 117), or is absent.

In the composition according to any one of the specific embodiments, it is characterized in that the peptide includes an amino acid sequence selected from the group consisting of SEQ ID NOS: 1 to 102.

In the composition according to any one of the specific embodiments, the peptide is characterized in that a ring is formed between the 16^{th} and 20^{th} amino acids from the N-terminus of the general formulae.

In the composition according to any one of the specific embodiments, the peptide is characterized in that the C-terminus of the peptide is amidated.

In the composition according to any one of the specific embodiments, it is characterized in that the pharmaceutical composition has at least one of the effects on increasing LDL absorption, inhibiting the activity of 3-hydroxy-3-methylglutaryl-CoA reductase (HMGCR), and promoting fatty acid degradation.

In the composition according to any one of the specific embodiments, after 24 to 48 hours from administration of the pharmaceutical composition, the activity of 3-hydroxy-3-methylglutaryl-CoA reductase (HMGCR) is less than 50%.

In the composition according to any one of the specific embodiments, the pharmaceutical composition reduces the LDL level and the triglyceride level in blood.

Another aspect of the present invention provides a pharmaceutical composition for preventing or treating hyperlipidemia including a conjugate including the peptide.

In a specific embodiment, it is characterized in that the conjugate is represented by Chemical Formula 1 below:

[Chemical Formula 1] X-Lₐ-F

In Chemical Formula 1,
X is the peptide with activity to a glucagon receptor, a GLP-1 receptor, and a GIP receptor,
L is polyethylene glycol, where a is 0 or a natural number, and when a is 2 or more, each L is independent, and
F is an immunoglobulin Fc region.

In another specific embodiment, it is characterized in that the peptide is a peptide including an amino acid sequence represented by General Formula 1 below:

Xaa1-Xaa2-Xaa3-Gly-Thr-Phe-Xaa7-Ser-Asp-Xaa10-Ser-Xaa12-Xaa13 -Xaa 14-Xaa 15-Xaa 16-Xaa 17-Xaa 18-Xaa 19-Xaa20-Xaa21 -Phe-Xaa23-Xaa24-Trp-Leu-Xaa27-Xaa28-Xaa29-Xaa30-R1 (General Formula 1, SEQ ID NO: 103)

In General Formula 1 above,
Xaa1 is histidine (His, H), 4-imidazoacetyl (CA), or tyrosine (Tyr, Y),
Xaa2 is glycine (Gly, G), α-methyl-glutamic acid, or aminoisobutyric acid (Aib),
Xaa3 is glutamic acid (Glu, E) or glutamine (Gln, Q),
Xaa7 is threonine (Thr, T) or isoleucine (Ile, I),
Xaa10 is leucine (Leu, L), tyrosine (Tyr, Y), lysine (Lys, K), cysteine (Cys, C), or valine (Val, V),
Xaa12 is lysine (Lys, K), serine (Ser, S), or isoleucine (Ile, I),
Xaa13 is glutamine (Gln, Q), tyrosine (Tyr, Y), alanine (Ala, A), or cysteine (Cys, C),
Xaa14 is leucine (Leu, L), methionine (Met, M), or tyrosine (Tyr, Y),
Xaa15 is cysteine (Cys, C), aspartic acid (Asp, D), glutamic acid (Glu, E), or leucine (Leu, L),
Xaa16 is glycine (Gly, G), glutamic acid (Glu, E), or serine (Ser, S),
Xaa17 is glutamine (Gln, Q), arginine (Arg, R), isoleucine (lie, I), glutamic acid (Glu, E), cysteine (Cys, C), or lysine (Lys, K),
Xaa18 is alanine (Ala, A), glutamine (Gln, Q), arginine (Arg, R), or histidine (His, H),
Xaa19 is alanine (Ala, A), glutamine (Gin, Q), cysteine (Cys, C), or valine (Val, V),
Xaa20 is lysine (Lys, K), glutamine (Gln, Q), or arginine (Arg, R),
Xaa21 is glutamic acid (Glu, E), glutamine (Gln, Q), leucine (Leu, L), cysteine (Cys, C), or aspartic acid (Asp, D),
Xaa23 is isoleucine (Ile, I) or valine (Val, V),
Xaa24 is alanine (Ala, A), glutamine (Gin, Q), cysteine (Cys, C), asparagine (Asn, N), aspartic acid (Asp, D), or glutamic acid (Glu, E),
Xaa27 is valine (Val, V), leucine (Leu, L), lysine (Lys, K), or methionine (Met, M),
Xaa28 is cysteine (Cys, C), lysine (Lys, K), alanine (Ala, A), asparagine (Asn, N), or aspartic acid (Asp, D),
Xaa29 is cysteine (Cys, C), glycine (Gly, G), glutamine (Gin, Q), threonine (Thr, T), glutamic acid (Glu, E), or histidine (His, H),
Xaa30 is cysteine (Cys, C), glycine (Gly, G), lysine (Lys, K), or histidine (His, H), or is absent, and
R1 is cysteine (Cys, C), GKKNDWKHNIT (SEQ ID NO: 106), m-SSGAPPPS-n (SEQ ID NO: 107), or m-SSGQPPPS-n (SEQ ID NO: 108), or is absent,
wherein m is -Cys-, -Pro-, or -Gly-Pro-, and
n is -Cys-, -Gly-, -Ser-, or -His-Gly-, or is absent.

In the composition according to any one of the specific embodiments, it is characterized in that the pharmaceutical composition includes a conjugate in which the peptide represented by General Formula 1 above is linked to the immunoglobulin Fc region via polyethylene glycol.

In the composition according to any one of the specific embodiments, it is characterized in that the pharmaceutical composition includes at least one of the effects on increasing LDL absorption, inhibiting the activity of 3-hydroxy-3-methylglutaryl-CoA reductase (HMGCR), and promoting fatty acid degradation.

In the composition according to any one of the specific embodiments, after 24 to 48 hours from administration of the pharmaceutical composition, the activity of 3-hydroxy-3-methylglutaryl-CoA reductase (HMGCR) is less than 50%.

In the composition according to any one of the specific embodiments, the pharmaceutical composition reduces the LDL level and the triglyceride level in blood.

Another aspect of the present invention provides a method of preventing or treating hyperlipidemia including administering the peptide, the conjugate thereof, or the composition including the same to an individual in need thereof.

Another aspect of the present invention provides a use of the peptide, the conjugate thereof, or the composition including the same for preparation of medicaments for preventing or treating hyperlipidemia.

### [Advantageous Effects]

A long-acting conjugate of the triple agonist of the present invention has activity to a glucagon receptor, a glucagon-like peptide-1 (GLP-1) receptor, and a glucose-dependent insulinotropic polypeptide (GIP) receptor, and is thereby applicable to a therapeutic agent of hyperlipidemia.

### [Brief Description of Drawings]

FIG. 1 is a graph illustrating *in vivo* therapeutic effects of a long-acting conjugate of a triple agonist of the present invention on hyperlipidemia.
FIG. 2 is a graph illustrating increases in LDL absorption by a long-acting conjugate of a triple agonist of the present invention.
FIG. 3 is a graph illustrating inhibition of 3-hydroxy-3-methylglutaryl-CoA reductase (HMGCR) activity by a long-acting conjugate of a triple agonist of the present invention.
FIG. 4 is a graph illustrating effects of a long-acting conjugate of a triple agonist of the present invention on promoting fatty acid degradation.

### [Best Mode]

Hereinafter, the present invention will be described in detail.

Meanwhile, each of the descriptions and embodiments disclosed herein may be applied to describe different descriptions and embodiments. That is, all of the combinations of various factors disclosed herein belong to the scope of the present invention. Furthermore, the scope of the present invention should not be limited by the detailed descriptions provided hereinbelow.

Throughout the specification, not only the conventional one-letter and three-letter codes for naturally occurring amino acids, but also those three-letter codes generally allowed for other amino acids, such as α-aminoisobutyric acid (Aib), N-methylglycine (Sar), and α-methyl-glutamic acid are used. In addition, the amino acids mentioned herein are abbreviated according to the nomenclature rules of the IUPAC-IUB as follows.

| | | | |
|---|---|---|---|
| alanine | Ala, A | arginine | Arg, R |
| asparagine | Asn, N | aspartic acid | Asp, D |
| cysteine | Cys, C | glutamic acid | Glu, E |
| glutamine | Gln, Q | glycine | Gly, G |
| histidine | His, H | isoleucine | Ile, I |
| leucine | Leu, L | lysine | Lys, K |
| methionine | Met, M | phenylalanine | Phe, F |
| proline | Pro, P | serine | Ser, S |
| threonine | Thr, T | tryptophan | Trp, W |
| tyrosine | Tyr, Y | valine | Val, V |

An aspect of the present invention provides a pharmaceutical composition for preventing or treating hyperlipidemia including a peptide with activity to a glucagon receptor, a glucagon-like peptide-1 (GLP-1) receptor, and a glucose-dependent insulinotropic polypeptide (GIP) receptor.

It is characterized in that the triple agonist of the present invention has therapeutic or preventive effects on hyperlipidemia. In the present invention, unlike dyslipidemia, in which the levels of cholesterol (low-density lipoprotein (LDL) cholesterol and high-density lipoprotein (HDL) cholesterol) and triglycerides (which are not cholesterol but a type of lipid) in blood are outside of normal ranges (including both increases and decreases), and hypercholesterolemia, in which a total cholesterol level in blood is high, hyperlipidemia is a general term for diseases in which one of the LDL cholesterol level and the triglyceride level or both are high in blood, and is distinguished from dyslipidemia and hypercholesterolemia. For example, in the case where the LDL level exceeds 130 mg/dL and/or the triglyceride level exceeds 200 mg/dL, it may correspond to hyperlipidemia.

**Table 1**

| Category | Cholesterol | | Triglycerides |
|---|---|---|---|
| | LDL Cholesterol | HDL Cholesterol | |
| Hyperlipidemia | excess of normal range | - | excess of normal range |
| Hypercholesterolemia | excess of normal range | | - |
| Dyslipidemia | excess of or below normal range | | |

In humans, the normal range of LDL cholesterol is 130 mg/dL or less, the normal range of HDL cholesterol is 60 mg/dL or more, specifically 40 mg/dL or more in men, and 50 mg/dL or more in women; and the normal range of triglycerides is 200 mg/dL or less. When a person has level(s) outside of the normal range(s) described above, it can be understood that the person has the disease(s) described in Table 1 above.

The conditions required for the treatment of hyperlipidemia are distinguished from those required for the treatment of hypercholesterolemia and dyslipidemia in that a remarkably excellent effect for reducing the levels of LDL and triglycerides is required for the effective treatment of hyperlipidemia, as shown in Table 1 above.

In this regard, the triple agonist of the present invention is characterized in that it can reduce the levels of LDL cholesterol and/or triglycerides in blood.

The term "peptide with activity to a glucagon receptor, a GLP-1 receptor, and a GIP receptor" may be used interchangeably with the triple agonist.

The peptide includes various substances, e.g., peptides, with a significant level of activity to the glucagon, GLP-1, and GIP receptors.

Although not particularly limited thereto, the triple agonist with a significant level of activity to the glucagon, GLP-1, and GIP receptors may exhibit *in vitro* activity of 0.1% or more, 1% or more, 2% or more, 3% or more, 4% or more, 5% or more, 6% or more, 7% or more, 8% or more, 9% or more, 10% or more, 20% or more, 30% or more, 40% or more, 50% or more, 60% or more, 70% or more, 80% or more, 90% or more, or 100% or more to at least one of the glucagon, GLP-1, and GIP receptors, specifically two or more receptors, more specifically all three receptors, compared to native ligands of the corresponding receptors (native glucagon, native GLP-1, and native GIP).

A method described below in Experimental Example 1 may be used to measure the *in vitro* activity of the triple agonist, without being limited thereto.

Meanwhile, it is characterized in that the peptide has the ability, specifically a significant level of the ability, to activate one or more, two or more, specifically all three of i) to iii) below:
i) the GLP-1 receptor; ii) the glucagon receptor; and iii) the GIP receptor.

In this regard, for example, the activating of the receptor indicates that the peptide has *in vitro* activity of 0.1% or more, 1% or more, 2% or more, 3% or more, 4% or more, 5% or more, 6% or more, 7% or more, 8% or more, 9% or more, 10% or more, 20% or more, 30% or more, 40% or more, 50% or more, 60% or more, 70% or more, 80% or more, 90% or more, or 100% or more to the receptors, compared to the native ligands. However, the present invention is not limited thereto.

The composition according to the present invention may have at least one of the effects on increasing LDL absorption, inhibiting cholesterol synthesis, and promoting fatty acid degradation by including the isolated peptide with activity to a glucagon receptor, a glucagon-like peptide-1 (GLP-1) receptor, and a glucose-dependent insulinotropic polypeptide (GIP) receptor, thereby having excellent therapeutic effects on hyperlipidemia. For example, after 24 to 48 hours from administration of the composition, the activity of 3-hydroxy-3-methylglutaryl-CoA reductase (HMGCR) may be less than 50%.

Specifically, after 24 hours or more from the administration of the composition to an individual (e.g., 24 to 48 hours), the activity of 3-hydroxy-3-methylglutaryl-CoA reductase (HMGCR) may be less than 50%, less than 40%, or less than 30%, and this suggests that the *in vivo* cholesterol synthesis of an individual may be inhibited by the composition according to the present invention and thereby provide a therapeutic effect for hyperlipidemia.

Additionally, the composition according to the present invention can reduce the LDL level and the triglyceride level in blood and thereby provide an excellent therapeutic effect specifically for hyperlipidemia.

In addition, the peptide may have an increased *in vivo* half-life compared to one of the native GLP-1, native glucagon, and native GIP, without being limited thereto.

Although not particularly limited thereto, the peptide may be one which is not naturally occurring.

Specifically, the isolated peptide may be an analog of native glucagon, without being limited thereto.

The native glucagon analog of the present invention may include a peptide having at least one difference in the amino acid sequence of native glucagon, a peptide having an amino acid sequence modified from that of the native glucagon, and mimics of native glucagon.

Meanwhile, although not particularly limited thereto, the native glucagon may have the following amino acid sequence:

His-Ser-Gln-Gly-Thr-Phe-Thr-Ser-Asp-Tyr-Ser-Lys-Tyr-Leu-Asp-Ser-Arg-Arg-Ala-Gln-Asp-Phe-Val-Gln-Trp-Leu-Met Asn-Thr (SEQ ID NO: 118)

Specifically, the peptide may be an analog of native glucagon in which one or more amino acids of the amino acid sequence of native glucagon are varied by substitution, addition, deletion, modification, and any combination thereof, without being limited thereto.

In addition, the substitution of amino acids includes substitution with a different amino acid or a non-native compound.

Also, the addition may occur at the N-terminus and/or C-terminus of the peptide. Meanwhile, a length of the added amino acids is not particularly limited, but 1 or more, 2 or more, 3 or more, 4 or more, 5 or more, 6 or more, 7 or more, 8 or more, 9 or more, 10 or more, or 11 or more amino acids may be added thereto, or in a wide range, a polypeptide may be added thereto, without being limited thereto.

More specifically, the glucagon analog may be a peptide in which 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, or 20 amino acids selected from the group consisting of the 1^{st}, 2^{nd}, 3^{rd}, 7^{th}, 10^{th}, 12^{th}, 13^{th}, 14^{th}, 15^{th}, 16^{th}, 17^{th}, 18^{th}, 19^{th}, 20^{th}, 21^{st}, 23^{rd}, 24^{th}, 27^{th}, 28^{th}, and 29^{th} amino acids of the amino acid sequence of native glucagon are substituted with a different amino acid, and also independently or additionally, 1 or more, 2 or more, 3 or more, 4 or more, 5 or more, 6 or more, 7 or more, 8 or more, 9 or more, 10 or more, or 11 or more amino acids are added to the C-terminus thereof, without being limited thereto.

Even more specifically, the glucagon analog may be a peptide in which 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, or 19 amino acids selected from the group consisting of the 1^{st}, 2^{nd}, 3^{rd}, 10^{th}, 12^{th}, 13^{th}, 14^{th}, 15^{th}, 16^{th}, 17^{th}, 18^{th}, 19^{th}, 20^{th}, 21^{st}, 23^{rd}, 24^{th}, 27^{th}, 28^{th}, and 29^{th} amino acids of the amino acid sequence of native glucagon are substituted with a different amino acid, and also independently or additionally, 1 or more, 2 or more, 3 or more, 4 or more, 5 or more, 6 or more, 7 or more, 8 or more, 9 or more, 10 or more, or 11 or more amino acids are added to the C-terminus thereof, without being limited thereto.

Even more specifically, the glucagon analog may be a peptide in which 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, or 17 amino acids selected from the group consisting of the 1^{st}, 2^{nd}, 3^{rd}, 10^{th}, 13^{th}, 14^{th}, 15^{th}, 16^{th}, 17^{th}, 18^{th}, 19^{th}, 20^{th}, 21^{st}, 23^{rd}, 24^{th}, 28^{th}, and 29^{th} amino acids of the amino acid sequence of native glucagon are substituted with a different amino acid, and also independently or additionally, 1 or more, 2 or more, 3 or more, 4 or more, 5 or more, 6 or more, 7 or more, 8 or more, 9 or more, 10 or more, or 11 or more amino acids are added to the C-terminus thereof, without being limited thereto.

Even more specifically, the glucagon analog may be a peptide in which 1 or more, 2 or more, 3 or more, 4 or more, 5 or more, 6 or more, 7 or more, 8 or more, 9 or more, 10 or more, 11 or more, 12 or more, 13 or more, or 14 amino acids selected from the group consisting of the 1^{st}, 2^{nd}, 13^{th}, 16^{th}, 17^{th}, 18^{th}, 19^{th}, 20^{th}, 21^{st}, 23^{rd}, 24^{th}, 27^{th}, 28^{th}, and 29^{th} amino acids of the amino acid sequence of native glucagon are substituted with a different amino acid, and also independently or additionally, 1 or more, 2 or more, 3 or more, 4 or more, 5 or more, 6 or more, 7 or more, 8 or more, 9 or more, 10 or more, or 11 or more amino acids are added to the C-terminus thereof, without being limited thereto.

The amino acid introduced into the native glucagon may be selected from the group consisting of tyrosine, α-methyl-glutamic acid, Aib, methionine, glutamic acid, histidine, lysine, leucine, isoleucine, glutamine, valine, glycine, alanine, cysteine, serine, alanine, aspartic acid, and arginine, without being limited thereto.

For example, the added amino acid sequence may include at least one amino acid sequence derived from the amino acid sequences of native GLP-1, native GIP, or native exendin-4.

The glucagon analog or triple agonist may include an intramolecular bridge (e.g., covalent crosslinking or non-covalent crosslinking), and specifically is in a form including a ring, for example, in a form where a ring is formed between the 16^{th} and 20^{th} amino acids of the glucagon analog or triple agonist, without being limited thereto.

Non-limiting examples of the ring may include a lactam bridge (or a lactam ring).

In addition, the glucagon analog or triple agonist includes all of those modified to include a ring or include an amino acid capable of forming a ring at a target position.

For example, the glucagon analog or triple agonist may be one where a pair of 16^{th} and 20^{th} amino acids are substituted with glutamic acid or lysine capable of forming a ring, but is not limited thereto.

The ring may be formed between amino acid side chains in the glucagon analog or triple agonist, e.g., a lactam ring may be formed between a side chain of lysine and a side chain of glutamic acid, but is not limited thereto.

Examples of the glucagon analog prepared by combination of these methods may include peptides having activities to the glucagon receptor, the GLP-1 receptor, and the GIP receptor having at least one different amino acid from that of native glucagon, from which an α-carbon of the amino acid residue of the N-terminus is removed, but are not limited thereto. The analog of native glucagon according to the present invention may be prepared by combining various methods used to prepare analogs.

Also, although not particularly limited thereto, in the peptide of the present invention, some amino acids may be substituted with different amino acids or a non-native compound to avoid recognition by an agonist-degrading enzyme for increasing *in vivo* half-life.

Specifically, the peptide may be one having an increased *in vivo* half-life by avoiding recognition by the agonist-degrading enzyme via substitution of the 2^{nd} amino acid in the amino acid sequence of the triple agonist, but any substitution or modification of amino acids to avoid recognition by the agonist-degrading enzyme in living organisms may be used without limitation.

In addition, such variation for the preparation of the analogues of native glucagon include variation using L-type or D-type amino acids and/or non-native amino acid; and/or variation of a native sequence, for example, variation of a side-chain functional group, intramolecular covalent bond, such as ring formation between side chains, methylation, acylation, ubiquitination, phosphorylation, aminohexanation, and biotinylation.

In addition, the variation includes all of those where one or more amino acids are added to the amino and/or carboxy terminus of native glucagon.

The amino acids substituted or added may be not only the 20 amino acids commonly found in human proteins but also atypical amino acids or those which are not naturally occurring. Commercial sources of atypical amino acids may include Sigma-Aldrich, ChemPep Inc., and Genzyme Pharmaceuticals. The peptides including theses amino acids and typical peptide sequences may be synthesized and purchased from commercial suppliers, e.g., American Peptide Company, Bachem, or Anygen (Korea).

Amino acid derivatives may also be obtained in the same manner; for example, 4-imidazoacetic acid may be used.

In addition, the peptide according to the present invention may be in a varied form where the N-terminus and/or C-terminus is chemically modified or protected by organic groups, or amino acids may be added to the termini of the peptide, for protection from proteases *in vivo* while increasing stability thereof.

Particularly, since the N- and C-termini of chemically synthesized peptides are electrically charged, the N-terminus may be acetylated and/or the C-terminus may be amidated to remove the charges, but the embodiment is not limited thereto.

In addition, the peptide according to the present invention includes all of those in the form of the peptide itself, a salt thereof (e.g., a pharmaceutically acceptable salt of the peptide), or a solvate thereof. Also, the peptide may be in any pharmaceutically acceptable form.

The type of the salt is not particularly limited. However, the salt is preferably in a form safe and effective to an individual, e.g., a mammal, without being limited thereto.

The term "pharmaceutically acceptable" refers to a substance that may be effectively used for the intended use within the scope of a pharmaco-medical decision without inducing excessive toxicity, irritation, allergic responses, and the like.

As used herein, the term "pharmaceutically acceptable salt" refers to a salt derived from a pharmaceutically acceptable inorganic acid, organic acid, or base. Examples of a suitable acid may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, and benzenesulfonic acid. Examples of the salt derived from a suitable base may include alkali metals such as sodium and potassium, alkaline earth metals such as magnesium, and ammonium.

In addition, as used herein, the term "solvate" refers to a complex of the peptide or a salt thereof according to the present invention and a solvent molecule.

In another specific embodiment of the present invention, the peptide may include an amino acid sequence represented by General Formula 1 below.

Xaa1-Xaa2-Xaa3-Gly-Thr-Phe-Xaa7-Ser-Asp-Xaa10-Ser-Xaa12-Xaa1 3-Xaa 14-Xaa15-Xaa16-Xaa17-Xaa18-Xaa 19-Xaa20-Xaa21 -Phe-Xaa23-Xaa24 -Trp-Leu-Xaa27-Xaa28-Xaa29-Xaa30-R1 (General Formula 1, SEQ ID NO: 103)

In General Formula 1 above, Xaa1 is histidine, 4-imidazoacetyl, or tyrosine, Xaa2 is glycine, α-methyl-glutamic acid, or Aib, Xaa3 is glutamic acid or glutamine, Xaa7 is threonine or isoleucine, Xaa10 is leucine, tyrosine, lysine, cysteine, or valine, Xaa12 is lysine, serine, or isoleucine, Xaa13 is glutamine, tyrosine, alanine, or cysteine, Xaa14 is leucine, methionine, or tyrosine, Xaa15 is cysteine, aspartic acid, glutamic acid, or leucine, Xaa16 is glycine, glutamic acid, or serine, Xaa17 is glutamine, arginine, isoleucine, glutamic acid, cysteine, or lysine, Xaa18 is alanine, glutamine, arginine, or histidine, Xaa19 is alanine, glutamine, cysteine, or valine, Xaa20 is lysine, glutamine, or arginine, Xaa21 is glutamic acid, glutamine, leucine, cysteine, or aspartic acid, Xaa23 is isoleucine or valine, Xaa24 is alanine, glutamine, cysteine, asparagine, aspartic acid, or glutamic acid, Xaa27 is valine, leucine, lysine, or methionine, Xaa28 is cysteine, lysine, alanine, asparagine, or aspartic acid, Xaa29 is cysteine, glycine, glutamine, threonine, glutamic acid, or histidine, Xaa30 is cysteine, glycine, lysine, or histidine or is absent, and
R1 is cysteine, GKKNDWKHNIT (SEQ ID NO: 106), m-SSGAPPPS-n (SEQ ID NO: 107), or m-SSGQPPPS-n (SEQ ID NO: 108), or is absent,
wherein m is -Cys-, -Pro-, or -Gly-Pro-, and n is -Cys-, -Gly-, -Ser-, or -His-Gly-, or is absent.

Examples of the triple agonist may include a peptide including an amino acid sequence selected from the group consisting of SEQ ID NOS: 1 to 102 and a peptide (essentially) consisting of an amino acid sequence selected from the group consisting of SEQ ID NOS: 1 to 102, without being limited thereto.

Also, although described as "a peptide consisting of a particular SEQ ID NO:" in the present invention, it does not exclude a mutation that may occurring naturally or by addition of a meaningless sequence upstream or downstream of the amino acid sequence of the SEQ ID NO, or a silent mutation thereof, as long as the peptide has activity identical or equivalent to that of the peptide consisting of the amino acid sequence, and even when such sequence addition or mutation is present, it obviously belongs to the scope of the present invention.

Descriptions given above may also be applied to other specific embodiments or aspects of the present invention, without being limited thereto.

Specifically, in General Formula 1 above, Xaa14 may be leucine or methionine, and Xaa15 may be cysteine, aspartic acid, or leucine.

Examples of the peptide may include a peptide including or (essentially) consisting of an amino acid sequence selected from the group consisting of SEQ ID NOS: 1 to 12, 14 to 17, and 21 to 102, without being limited thereto.

The peptide may be one capable of significantly activating the glucagon receptor, the GLP-1 receptor, and the GIP receptor, without being limited thereto. Specifically, the peptide may be one capable of significantly activating the GLP-1, and additionally, the glucagon receptor and/or the GIP receptor, without being limited thereto.

More specifically, in General Formula 1 above, Xaa2 is glycine, α-methyl-glutamic acid, or Aib, Xaa7 is threonine, Xaa10 is tyrosine, cysteine, or valine, Xaa12 is lysine or isoleucine, Xaa13 is tyrosine, alanine, glutamine, or cysteine, Xaa14 is leucine, cysteine, or methionine, Xaa15 is cysteine, leucine, glutamic acid, or aspartic acid, Xaa17 is glutamine, arginine, isoleucine, cysteine, glutamic acid, or lysine, Xaa18 is alanine, glutamine, arginine, or histidine, Xaa19 is alanine, glutamine, valine, or cysteine, Xaa20 is lysine, arginine, or glutamine, Xaa21 is glutamic acid, glutamine, leucine, cysteine, or aspartic acid, Xaa23 is isoleucine or valine, Xaa24 is cysteine, alanine, glutamine, asparagine, glutamic acid, or aspartic acid, and Xaa27 is leucine or lysine, without being limited thereto.

Evan more specifically, in General Formula 1 above, Xaa2 is glycine, α-methyl-glutamic acid, or Aib, Xaa7 is threonine, Xaa10 is tyrosine, cysteine, or valine, Xaa12 is lysine or isoleucine, Xaa13 is tyrosine, alanine, or cysteine, Xaa14 is leucine or methionine, Xaa15 is cysteine or aspartic acid, Xaa17 is glutamine, arginine, isoleucine, cysteine, or lysine, Xaa18 is alanine, arginine, or histidine, Xaa19 is alanine, glutamine, or cysteine, Xaa20 is lysine or glutamine, Xaa21 is glutamic acid, cysteine, or aspartic acid, Xaa23 is valine, Xaa24 is alanine, glutamine, cysteine, asparagine, or aspartic acid, and Xaa27 is leucine or lysine, without being limited thereto.

Even more specifically, in General Formula 1 above, Xaa2 is α-methyl-glutamic acid or Aib, Xaa7 is threonine, Xaa10 is tyrosine or cysteine, Xaa12 is lysine or isoleucine, Xaa13 is tyrosine, alanine, or cysteine, Xaa14 is leucine or methionine, Xaa15 is cysteine or aspartic acid, Xaa16 is glutamic acid, Xaa17 is arginine, isoleucine, cysteine, or lysine, Xaa18 is alanine, arginine, or histidine, Xaa19 is alanine, glutamine, or cysteine, Xaa20 is lysine or glutamine, Xaa21 is glutamic acid or aspartic acid, Xaa23 is valine, Xaa24 is glutamine, asparagine, or aspartic acid, Xaa27 is leucine, and Xaa28 is cysteine, alanine, asparagine, or aspartic acid.

Specifically, in General Formula 1 above, Xaa1 is histidine or 4-imidazoacetyl, Xaa2 is α-methyl-glutamic acid or Aib, Xaa3 is glutamine, Xaa7 is threonine, Xaa10 is tyrosine, Xaa12 is isoleucine, Xaa13 is alanine or cysteine, Xaa14 is methionine, Xaa15 is aspartic acid, Xaa16 is glutamic acid, Xaa17 is isoleucine or lysine, Xaa18 is alanine or histidine, Xaa19 is glutamine or cysteine, Xaa20 is lysine, Xaa21 is aspartic acid, Xaa23 is valine, Xaa24 is asparagine, Xaa27 is leucine, Xaa28 is alanine or asparagine, Xaa29 is glutamine or threonine, and Xaa30 is cysteine or lysine, or is absent.

More specifically, in General Formula 1 above, Xaa2 is glycine, α-methyl-glutamic acid, or Aib, Xaa3 is glutamine, Xaa7 is threonine, Xaa10 is tyrosine, cysteine, or valine, Xaa12 is lysine, Xaa13 is tyrosine, Xaa14 is leucine, Xaa15 is aspartic acid, Xaa16 is glycine, glutamic acid, or serine, Xaa17 is glutamine, arginine, cysteine, or lysine, Xaa18 is alanine, arginine, or histidine, Xaa19 is alanine or glutamine, Xaa20 is lysine or glutamine, Xaa21 is glutamic acid, cysteine, or aspartic acid, Xaa23 is valine, Xaa24 is alanine, glutamine, or cysteine, Xaa27 is leucine or lysine, and Xaa29 is glycine, glutamine, threonine, or histidine, without being limited thereto.

The peptide may be a peptide having significant ability to activate the GLP-1 receptor and the glucagon receptor and higher ability to activate the GIP receptor; a peptide having significant ability to activate the GLP-1 receptor, the glucagon receptor and the GIP receptor; or a peptide having significant ability to activate the GLP-1 receptor and the GIP receptor and higher ability to activate the glucagon receptor, without being limited thereto.

Examples of the peptide may include a peptide including or (essentially) consisting of an amino acid sequence selected from the group consisting of SEQ ID NOS: 8, 9, 21 to 37, 39, 42, 43, 49 to 61, 64 to 83, 85, 86, 88, 89, 91 to 93, and 95 to 102, without being limited thereto.

In a specific embodiment, the peptide may include an amino acid sequence represented by General Formula 2 below.

Xaa1-Xaa2-Gln-Gly-Thr-Phe-Thr-Ser-Asp-Xaa10-Ser-Lys-Xaa13-Xaa 14-Xaa 15-Xaa 16-Xaa 17-Xaa 18-Xaa 19-Xaa20-Xaa21 -Phe-Xaa23-Xaa24-Trp-Leu-Leu-Xaa28-Xaa29-Xaa30-Xaa31-Ser-Ser-Gly-Gln-Pro-Pro-Pro-Ser-Xaa4 0 (General Formula 2, SEQ ID NO: 104)

In General Formula 2 above, Xaa1 is 4-imidazoacetyl, histidine, or tyrosine; Xaa2 is glycine, α-methyl-glutamic acid, or Aib; Xaa10 is tyrosine or cysteine; Xaa13 is alanine, glutamine, tyrosine, or cysteine; Xaa14 is leucine, methionine, or tyrosine; Xaa15 is aspartic acid, glutamic acid, or leucine; Xaa16 is glycine, glutamic acid, or serine; Xaa17 is glutamine, arginine, isoleucine, glutamic acid, cysteine, or lysine; Xaa18 is alanine, glutamine, arginine, or histidine; Xaa19 is alanine, glutamine, cysteine, or valine; Xaa20 is lysine, glutamine, or arginine; Xaa21 is cysteine, glutamic acid, glutamine, leucine, or aspartic acid; Xaa23 is isoleucine or valine; Xaa24 is cysteine, alanine, glutamine, asparagine, or glutamic acid; Xaa28 is lysine, cysteine, asparagine, or aspartic acid; Xaa29 is glycine, glutamine, cysteine, or histidine; Xaa30 is cysteine, glycine, lysine, or histidine; Xaa31 is proline or cysteine; and Xaa40 is cysteine, or is absent.

More specifically, in General Formula 2 above, Xaa13 is alanine, tyrosine, or cysteine; Xaa15 is aspartic acid or glutamic acid; Xaa17 is glutamine, arginine, cysteine, or lysine; Xaa18 is alanine, arginine, or histidine; Xaa21 is cysteine, glutamic acid, glutamine, or aspartic acid; Xaa23 is isoleucine or valine; Xaa24 is cysteine, glutamine, or asparagine; Xaa28 is cysteine, asparagine, or aspartic acid; Xaa29 is glutamine, cysteine, or histidine; and Xaa30 is cysteine, lysine, or histidine.

Examples of the peptide may include a peptide including or (essentially) consisting of an amino acid sequence selected from the group consisting of SEQ ID NOS: 21, 22, 42, 43, 50, 64 to 77, and 95 to 102, more specifically an amino acid sequence selected from the group consisting of SEQ ID NOS: 21, 22, 42, 43, 50, 64 to 77, and 96 to 102, without being limited thereto.

In a specific embodiment, the peptide may include an amino acid sequence of General Formula 3 below.

Xaa1-Xaa2-Gln-Gly-Thr-Phe-Thr-Ser-Asp-Tyr-Ser-Lys-Xaa13-Leu-As p-Glu-Xaa17-Xaa18-Xaa19-Lys-Xaa21-Phe-Val-Xaa24-Trp-Leu-Leu-Xaa28-X aa29-Xaa30-Xaa31-Ser-Ser-Gly-Gln-Pro-Pro-Pro-Ser-Xaa40 (General Formula 3, SEQ ID NO: 105)

In General Formula 3 above, Xaa1 is histidine or tyrosine; Xaa2 is α-methyl-glutamic acid or Aib; Xaa13 is alanine, tyrosine or cysteine; Xaa17 is arginine, cysteine, or lysine; Xaa18 is alanine or arginine; Xaa19 is alanine or cysteine; Xaa21 is glutamic acid or aspartic acid; Xaa24 is glutamine or asparagine; Xaa28 is cysteine or aspartic acid; Xaa29 is cysteine, histidine, or glutamine; Xaa30 is cysteine or histidine; Xaa31 is proline or cysteine; and Xaa40 is cysteine, or is absent.

Examples of the peptide may be a peptide including or (essentially) consisting of an amino acid sequence selected from the group consisting of SEQ ID NOS: 21, 22, 42, 43, 50, 64 to 71, 75 to 77, and 96 to 102, without being limited thereto.

In addition, in General Formula 1 above, R1 is cysteine, GKKNDWKHNIT (SEQ ID NO: 106), CSSGQPPPS (SEQ ID NO: 109), GPSSGAPPPS (SEQ ID NO: 110), GPSSGAPPPSC (SEQ ID NO: 111), PSSGAPPPS (SEQ ID NO: 112), PSSGAPPPSG (SEQ ID NO: 113), PSSGAPPPSHG (SEQ ID NO: 114), PSSGAPPPSS (SEQ ID NO: 115), PSSGQPPPS (SEQ ID NO: 116), or PSSGQPPPSC (SEQ ID NO: 117), or is absent, without being limited thereto.

In addition, the peptide of the present invention may be synthesized, according to the length, by a method well known in the art, e.g., by an automatic peptide synthesizer, and may be produced by way of genetic engineering technology.

Specifically, the peptide of the present invention may be prepared by way of a standard synthesis method, a recombinant expression system, or any other method known in the art. Thus, the peptide according to the present invention may be synthesized by way of a plurality of methods including the following methods:
(a) a method of synthesizing a peptide in a stepwise or fragment-assembling manner by way of a solid-phase or liquid-phase method, followed by isolation and purification of a final peptide product;
(b) a method of expressing a nucleic acid construct encoding a peptide in a host cell and recovering an expression product from a host cell culture;
(c) a method of performing *in vitro* cell-free expression of a nucleic acid construct encoding a peptide and recovering an expression product therefrom; or
a method of obtaining peptide fragments by way of any combination of the methods (a), (b), and (c), obtaining the peptide by linking the peptide fragments, and then recovering the peptide.

In addition, the peptide with activity to the glucagon receptor, the GLP-1 receptor, and the GIP receptor may be in the form of a conjugate in which a biocompatible material capable of increasing *in vivo* half-life of the peptide is linked to the peptide with activity to the glucagon receptor, the GLP-1 receptor, and the GIP receptor. Throughout the specification, the biocompatible material may be used interchangeably with a carrier.

In the present invention, the conjugate of the peptide may exhibit long-lasting effects that are increased compared to those of the peptide not linked to the carrier, and throughout the specification, the conjugate is referred to as a "long-acting conjugate".

Meanwhile, the conjugate may be one which does not occur naturally.

Another aspect of the present invention provides a pharmaceutical composition for preventing or treating hyperlipidemia including a conjugate including a peptide with activity to a glucagon receptor, a glucagon-like peptide-1 (GLP-1) receptor, and a glucose-dependent insulinotropic polypeptide (GIP) receptor.

In a specific embodiment of the present invention, the conjugate is a conjugate represented by Chemical Formula 1 below:

[Chemical Formula 1] X-Lₐ-F

In Chemical Formula 1, X is a peptide with activity to a glucagon receptor, a GLP-1 receptor, and a GIP receptor; L is polyethylene glycol; a is 0 or a natural number, where when a is 2 or greater, each L is independent; and F is an immunoglobulin Fc region.

In the conjugate, X, i.e., the peptide with activity to the glucagon receptor, the GLP-1 receptor, and the GIP receptor, is as described above.

In the conjugate, F is a substance capable of increasing the half-life of X, *i.e.,* the peptide with activity to the glucagon receptor, the GLP-1 receptor, and the GIP receptor, corresponding to a component of a moiety constituting the conjugate of the present invention.

F and X may be linked to each other by a covalent chemical bond or a non-covalent chemical bond, and F and X may be linked to each other via L by a covalent chemical bond, a non-covalent chemical bond, or any combination thereof.

The immunoglobulin Fc region may specifically be an IgG Fc region, without being limited thereto.

In a specific embodiment of the present invention, F (immunoglobulin Fc region) may have a structure of a dimer consisting of two polypeptide chains, in which one end of L may be linked to only one of the two polypeptide chains, without being limited thereto.

At least one amino acid side chain in the peptide of the present invention may be attached to the biocompatible material in order to increase *in vivo* solubility and/or half-life, and/or increase bioavailability thereof. These modifications may reduce the clearance of therapeutic proteins and peptides.

The biocompatible material may be water-soluble (amphipathic or hydrophilic), non-toxic, and/or pharmaceutically acceptable.

F may be linked to X directly (i.e., a is 0 in Chemical Formula 1) or via a linker (L).

Specifically, L may be a polyethylene glycol linker, which is a non-peptidyl linker. As used herein, the term "polyethylene glycol linker" includes a biocompatible polymer in which two or more ethylene glycol repeat units are bound together. The repeat units are linked to each other through an arbitrary covalent bond, not a peptide bond. The polyethylene glycol linker may be one constitution which constitutes a moiety of the conjugate of the present invention, and it corresponds to L in Chemical Formula 1.

In Lₐ, a may be 1 or more, and when a is 2 or more, each L may be independent.

In addition, in a specific embodiment, the conjugate may be one in which F is linked to X by covalent bonds via a non-peptidyl linker including reactive groups at both ends respectively capable of bonding to F, specifically an immunoglobulin Fc region, and X, specifically a peptide drug.

Specifically, L (*i.e.,* a polyethylene glycol linker) may be a linker containing an ethylene glycol repeat unit (e.g., polyethylene glycol), without being limited thereto. In the present specification, the polyethylene glycol is a general term including all of the forms of homopolymers of ethylene glycol, PEG copolymers, and monomethyl-substituted PEG polymers (mPEG), without being particularly limited thereto. Additionally, those derivatives which are already known in the art and the derivatives that can easily be prepared at the technological level of those skilled in the art are included in the scope of the present invention.

The polyethylene glycol linker may be one which includes a functional group used for the preparation of a conjugate at an end while including an ethylene glycol repeat unit. The long-acting conjugate according to the present invention may be in the form in which X and F are linked through the functional group, without being limited thereto. In the present invention, the non-peptidyl linker may include two, or three or more functional groups, and each functional group may be the same as or different from each other, without being limited thereto.

Specifically, the linker may be polyethylene glycol (PEG) represented by Chemical Formula 2 below, without being limited thereto:

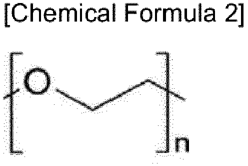

wherein n is 10 to 2,400, n is 10 to 480, or n is 50 to 250, without being limited thereto.

In the long-acting conjugate above, the PEG moiety may include not only the -(CH₂CH₂O)ₙ- structure, but also an oxygen atom interposed between a linking element and the -(CH₂CH₂O)ₙ- structure, without being limited thereto.

Additionally, in a specific embodiment, the long-acting conjugate may have a structure in which the peptide (X) of the present invention and an immunoglobulin fragment (F) are linked by a covalent bond via a linker (L) containing an ethylene glycol repeat unit, without being limited thereto.

The molecular weight of the non-peptide polymer may be in the range of greater than 0 kDa to 200 kDa, specifically about 1 kDa to about 100 kDa, about 1 kDa to about 50 kDa, about 1 kDa to about 30 kDa, about 2 kDa to about 30 kDa, about 1 kDa to about 20 kDa, more specifically about 3.4 kDa to about 10 kDa, and even more specifically about 3.4 kDa, without being limited thereto.

As used herein, the term "about" refers to a range including all of ± 0.5, ± 0.4, ± 0.3, ± 0.2, ± 0.1, etc., and it includes all of the values equivalent to those which come immediately after the term "about" or those in a similar range, without being limited thereto.

In addition to polyethylene glycol, any known derivatives thereof and any derivatives easily prepared by techniques known in the art also fall within the scope of the present invention.

In addition, the non-peptidyl linker of the present invention linked to the polypeptide corresponding to F may be not only a polymer of one type but also a combination of different types of polymers.

In a specific embodiment, both ends of the non-peptidyl linker may be linked to an amine or thiol group of F, e.g., the immunoglobulin Fc region, and an amine or thiol group of X, respectively.

Specifically, the non-peptidyl polymer may include reactive groups at both ends respectively linked to F (*e.g.,* immunoglobulin Fc region) and X, specifically an amine group of the N-terminus or lysine and a thiol group of cysteine of X or F (e.g., immunoglobulin Fc region), without being limited thereto.

In addition, the reactive groups of the non-peptidyl polymer capable of being linked to F, e.g., an immunoglobulin Fc region, and X may be selected from the group consisting of an aldehyde group, a maleimide group, and a succinimide derivative, but are not limited thereto.

In the above description, the aldehyde group may be a propionaldehyde group or a butyraldehyde group, without being limited thereto.

In the above description, the succinimide derivative may be succinimidyl valerate, succinimidyl methyl butanoate, succinimidyl methylpropionate, succinimidyl butanoate, succinimidyl propionate, N-hydroxysuccinimide, hydroxy succinimidyl, succinimidyl carboxymethyl, or succinimidyl carbonate, without being limited thereto.

The non-peptidyl linker may be linked to X and F via such reactive groups, without being limited thereto.

Also, a final product produced by reductive amination by aldehyde bonds is more stable than that formed by an amide bond. The aldehyde reactive group selectively reacts with the N-terminus at low pH while forming a covalent bond with a lysine residue at high pH, e.g., at a pH of 9.0.

In addition, the reactive groups of both ends of the non-peptidyl linker may be the same or different, for example, a maleimide group may be provided at one end and an aldehyde group, a propionaldehyde group, or a butyraldehyde group may be provided at the other end. However, the reactive groups are not particularly limited as long as F, specifically the immunoglobulin Fc region, and X may be linked to the respective ends of the non-peptide linker.

For example, the non-peptide linker may include a maleimide group at one end and an aldehyde group, a propionaldehyde group, or a butyraldehyde group at the other end, as reactive groups.

When polyethylene glycol having hydroxyl reactive groups at both ends is used as the non-peptide polymer, the long-acting protein conjugate according to the present invention may be prepared by activating the hydroxyl groups to various reactive groups by known chemical reactions, or using commercially available polyethylene glycol having modified reactive groups.

In a specific embodiment, the non-peptide polymer may be linked to a cysteine residue, more specifically a -SH group of cysteine, of X, without being limited thereto.

For example, the non-peptidyl polymer may be linked to a cysteine residue located at the 10^{th} position, a cysteine residue located at the 13^{th} position, a cysteine residue located at the 15^{th} position, a cysteine residue located at the 17^{th} position, a cysteine residue located at the 19^{th} position, a cysteine residue located at the 21^{st} position, a cysteine residue located at the 24^{th} position, a cysteine residue located at the 28^{th} position, a cysteine residue located at the 29^{th} position, a cysteine residue located at the 30^{th} position, a cysteine residue located at the 31^{st} position, a cysteine residue located at the 40^{th} position, or a cysteine residue located at the 41^{st} position of the peptide corresponding to X, without being limited thereto.

Specifically, a reactive group of the non-peptidyl polymer may be linked to the -SH group of the cysteine residue, and the reactive group is as described above. When maleimide-PEG-aldehyde is used, the maleimide group may be linked to the -SH group of X via a thioether bond, and the aldehyde group may be linked to the -NH₂ group of F, specifically the immunoglobulin Fc region, via reductive amination, but this is merely an example, and the present invention is not limited thereto.

Also, in the conjugate, a reactive group of the non-peptide polymer may be linked to the -NH₂ group located at the N-terminus of the immunoglobulin Fc region, but this is merely an example.

As used herein, the term "immunoglobulin Fc region" refers to a region including a heavy chain constant region 2 (CH2) and/or a heavy chain constant region 3 (CH3) excluding the heavy chain and light chain variable regions of the immunoglobulin. The immunoglobulin Fc region may be a component constituting a moiety of the conjugate of the present invention.

In the present specification, an Fc region includes not only the native sequence of an Fc fragment obtained by papain digestion of full-length immunoglobulin, but also a derivative of the native sequence of an Fc fragment (e.g., sequences in which one or more amino acid residues in the native sequence are modified by deletion (*e.g.,* deletion of a part of a hinge region), insertion, non-conservative or conservative substitution, or a combination thereof). In the present specification, the term "biocompatible material" or "carrier" may refer to the Fc region.

The F has a structure in which two polypeptide chains are linked by a disulfide bond, and may have a structure in which two polypeptide chains are linked through a nitrogen atom in only one of the two chains, without being limited thereto. The linkage through the nitrogen atom may be linked to the *epsilon*-N atom or the N-terminus amino group of lysine via reductive amination.

The reductive amination reaction refers to a reaction in which an amine group or amino group of a reactant reacts with an aldehyde of another reactant (i.e., a functional group capable of reductive amination) to produce an amine, and an amine bond is formed by a reduction reaction thereafter. The reductive amination reaction is a reaction of organic synthesis widely known in the art.

In an embodiment of the long-acting conjugate of the present invention, the long-acting conjugate may be one in which the immunoglobulin Fc region is linked to a linker through a N-terminus nitrogen atom thereof, without being limited thereto.

The immunoglobulin Fc region may include a hinge region in the heavy chain constant region, without being limited thereto.

In the present invention, the immunoglobulin Fc region may include a specific hinge sequence in the N-terminus.

As used herein, the term "hinge sequence" refers to a region which is located in the heavy chain and forms a dimer of the immunoglobulin Fc region through an inter-disulfide bond.

In the present invention, the hinge sequence may be a modified sequence in which part of the hinge sequence having the following amino acid sequence is deleted such that there is only one cysteine residue in the sequence, without being limited thereto:
Glu-Ser-Lys-Tyr-Gly-Pro-Pro-Cys-Pro-Ser-Cys-Pro (SEQ ID NO: 119).

The hinge sequence may be one in which the 8^{th} or 11^{th} cysteine residue in the hinge sequence of SEQ ID NO: 119 is deleted such that only one cysteine residue is included in the sequence. The hinge sequence of the present invention may consist of 3 to 12 amino acids, including only one cysteine residue, without being limited thereto. More specifically, the hinge sequence of the present invention may have the following sequences:
Glu-Ser-Lys-Tyr-Gly-Pro-Pro-Pro-Ser-Cys-Pro (SEQ ID NO: 120), Glu-Ser-Lys-Tyr-Gly-Pro-Pro-Cys-Pro-Ser-Pro (SEQ ID NO: 121), Glu-Ser-Lys-Tyr-Gly-Pro-Pro-Cys-Pro-Ser (SEQ ID NO: 122), Glu-Ser-Lys-Tyr-Gly-Pro-Pro-Cys-Pro-Pro (SEQ ID NO: 123), Lys-Tyr-Gly-Pro-Pro-Cys-Pro-Ser (SEQ ID NO: 124), Glu-Ser-Lys-Tyr-Gly-Pro-Pro-Cys (SEQ ID NO: 125), Glu-Lys-Tyr-Gly-Pro-Pro-Cys (SEQ ID NO: 126), Glu-Ser-Pro-Ser-Cys-Pro (SEQ ID NO: 127), Glu-Pro-Ser-Cys-Pro (SEQ ID NO: 128), Pro-Ser-Cys-Pro (SEQ ID NO: 129), Glu-Ser-Lys-Tyr-Gly-Pro-Pro-Ser-Cys-Pro (SEQ ID NO: 130), Lys-Tyr-Gly-Pro-Pro-Pro-Ser-Cys-Pro (SEQ ID NO: 131), Glu-Ser-Lys-Tyr-Gly-Pro-Ser-Cys-Pro (SEQ ID NO: 132), Glu-Ser-Lys-Tyr-Gly-Pro-Pro-Cys (SEQ ID NO: 133), Lys-Tyr-Gly-Pro-Pro-Cys-Pro (SEQ ID NO: 134), Glu-Ser-Lys-Pro-Ser-Cys-Pro (SEQ ID NO: 135), Glu-Ser-Pro-Ser-Cys-Pro (SEQ ID NO: 136), Glu-Pro-Ser-Cys (SEQ ID NO: 137), and Ser-Cys-Pro (SEQ ID NO: 138).

More specifically, the hinge sequence may be one which includes the amino acid sequence of SEQ ID NO: 129 (Pro-Ser-Cys-Pro) or SEQ ID NO: 138 (Ser-Cys-Pro), without being limited thereto.

In a more specific embodiment of the long-acting conjugate of the present invention, the N-terminus of the immunoglobulin Fc region within the conjugate is proline, and the conjugate is one in which the immunoglobulin Fc region is linked to a linker through a nitrogen atom of the proline.

In an aspect of the long-acting conjugate of the present invention, the immunoglobulin Fc region may be in the form of a dimer in which two chains of the immunoglobulin Fc region form a homodimer or heterodimer due to the presence of a hinge sequence therein. The conjugate of Chemical Formula 1 of the present invention may be in the form in which one end of the linker is linked to one chain of the dimeric immunoglobulin Fc region, without being limited thereto.

As used herein, the term "N-terminus" refers to the amino terminus of a protein or polypeptide, and it may include 1, 2, 3, 4, 5, 6, 7, 8, 9, or 10 or more amino acids from the most terminal end or the terminal end of the amino terminus. The immunoglobulin Fc region of the present invention may include a hinge sequence in the N-terminus, without being limited thereto.

Also, the immunoglobulin Fc region of the present invention may be an extended Fc region including a part of or the entirety of a heavy chain constant region 1 (CH1) and/or a light chain constant region 1 (CL1) excluding the heavy chain and the light chain variable regions of the immunoglobulin, as long as the immunoglobulin Fc region has substantially identical or enhanced effects compared to the native type. Also, the immunoglobulin Fc region may be a region from which a considerably long part of the amino acid sequence corresponding to the CH2 and/or CH3 is removed.

For example, the immunoglobulin Fc region of the present invention may include 1) CH1 domain, CH2 domain, CH3 domain, and CH4 domain, 2) CH1 domain and CH2 domain, 3) CH1 domain and CH3 domain, 4) CH2 domain and CH3 domain, 5) a combination of one or more domains selected from the CH1 domain, CH2 domain, CH3 domain, and CH4 domain and an immunoglobulin hinge region (or a part of the hinge region) or, 6) a dimer of each domain of the heavy chain constant region and the light chain constant region, without being limited thereto.

Also, in an embodiment of the long-acting conjugate of the present invention, the immunoglobulin Fc region F is a dimer consisting of two polypeptide chains, and the Fc region dimer F and X are covalently linked through one common linker containing an ethylene glycol repeat unit. In a specific embodiment of the aspect, X is covalently linked through a linker L to only one polypeptide chain of the two polypeptide chains of the Fc region dimer F. In a more specific embodiment of the aspect, in the one polypeptide chain between the two polypeptide chains of the Fc region dimer F to which X is linked, only one molecule of X is covalently linked through L. In the most specific embodiment of the aspect, F is a homodimer.

In another aspect of the long-acting conjugate of the present invention, it is possible that two molecules of X are symmetrically linked to one Fc region in a dimeric form. In this case, the immunoglobulin Fc region and X may be linked to each other via L. However, the embodiment is not limited to the above-described examples.

In addition, the immunoglobulin Fc region of the present invention includes not only the native amino acid sequence but also a sequence derivative thereof. The amino acid sequence derivative refers to an amino acid sequence having at least one different amino acid residue by deletion, addition, non-conservative or conservative substitution, or any combination thereof.

For example, in the case of IgG Fc, amino acid residues at positions 214 to 238, 297 to 299, 318 to 322, or 327 to 331, which are known to be important in binding, may be used as suitable sites for modification.

Also, various other types of derivatives may be prepared, for example, by removing a region capable of forming a disulfide bond, removing several amino acids from the N-terminus of the native Fc, or adding a methionine residue to the N-terminus of the native Fc. Also, to remove effector functions, a complement-binding site, e.g., a C1q-binding site, may be removed, or an antibody-dependent cell-mediated cytotoxicity (ADCC) site may be removed. Techniques of preparing these sequence derivatives of the immunoglobulin Fc region are disclosed in International Patent Publication Nos. WO 97/34631, WO 96/32478, *etc.*

Amino acid exchanges in proteins and peptides which do not alter the activity thereof are well known in the art (H. Neurath, R. L. Hill, The Proteins, Academic Press, New York, 1979). The most commonly occurring exchanges are exchanges between amino acid residues, e.g., Ala/Ser, Val/lie, AspIGlu, ThrISer, Ala/Gly, Ala/Thr, Ser/Asn, Ala/Val, SerIGly, Thy/Phe, Ala/Pro, Lys/Arg, AsplAsn, Leu/lie, Leu/Val, Ala/Glu, and Asp/Gly. If required, the Fc region may be modified by phosphorylation, sulfation, acrylation, glycosylation, methylation, farnesylation, acetylation, amidation, and the like.

The above-described Fc derivatives may have biological activity identical to that of the Fc region of the present invention and improved structural stability against heat, pH, or the like.

In addition, the Fc region may be obtained from native forms isolated *in vivo* from humans or animals such as cows, goats, pigs, mice, rabbits, hamsters, rats, or guinea pigs, or may be recombinants or derivatives thereof, obtained from transformed animal cells or microorganisms. In this regard, the Fc region may be obtained from a native immunoglobulin by isolating whole immunoglobulin from a living body of a human or animal and treating the isolated immunoglobulin with a protease. When the whole immunoglobulin is treated with papain, it is cleaved into Fab and Fc regions, whereas when the whole immunoglobulin is treated with pepsin, it is cleaved into pF'c and F(ab)₂ fragments. Fc or pF'c may be isolated therefrom using size-exclusion chromatography or the like. In a more specific embodiment, a human-derived Fc region is a recombinant immunoglobulin Fc region obtained from a microorganism.

In addition, the immunoglobulin Fc region may have natural glycans or increased or decreased glycans compared to the natural type, or may be in a deglycosylated form. The increase, decrease, or removal of glycans of the immunoglobulin Fc may be achieved by way of any methods commonly used in the art such as a chemical method, an enzymatic method, and a genetic engineering method using a microorganism. In this regard, the immunoglobulin Fc region obtained by removing glycans shows a significant decrease in binding affinity to a complement c1q and a decrease in or loss of antibody-dependent cytotoxicity or complement-dependent cytotoxicity, and thus unnecessary immune responses are not induced thereby in living organisms. Based thereon, a deglycosylated or aglycosylated immunoglobulin Fc region may be more suitable as a drug carrier in view of the objects of the present invention.

As used herein, the term "deglycosylation" refers to an Fc region from which glycan is removed using an enzyme, and the term "aglycosylation" refers to an Fc region that is not glycosylated and produced in prokaryotes, more specifically *E*. *coli.*

Meanwhile, the immunoglobulin Fc region may be derived from humans or animals such as cows, goats, pigs, mice, rabbits, hamsters, rats, or guinea pigs. In a more specific embodiment, the immunoglobulin Fc region may be derived from humans.

In addition, the immunoglobulin Fc region may be derived from IgG, IgA, IgD, IgE, or IgM, or any combination or hybrid thereof. In a more specific embodiment, the immunoglobulin Fc region is derived from IgG or IgM, which are the most abundant proteins in human blood, and in an even more specific embodiment, it is derived from IgG, which is known to enhance the half-lives of ligand-binding proteins. In a yet even more specific embodiment, the immunoglobulin Fc region is an IgG4 Fc region, and in the most specific embodiment, the immunoglobulin Fc region is an aglycosylated Fc region derived from human IgG4, without being limited thereto.

Additionally, in a specific embodiment, the immunoglobulin Fc region, being a human IgG4 fragment, may be in the form of a homodimer in which two monomers are linked through a disulfide bond (an inter-chain form) between cysteines, which are the 3^{rd} amino acid of each monomer. In particular, the homodimer has/or can have a disulfide bond between the cysteines at positions 35 and 95 and a disulfide bond between the cysteines at positions 141 and 199 in each monomer (i.e., two disulfide bonds (an intra-chain form)).

With respect to the number of amino acids, each monomer may consist of 221 amino acids, and the amino acids forming a homodimer may consist of a total of 442 amino acids, without being limited thereto. Specifically, the immunoglobulin Fc fragment may be one in which two monomers having the amino acid sequence of SEQ ID NO: 139 (consisting of 221 amino acids) form a homodimer through a disulfide bond between cysteines, which are the 3^{rd} amino acid of each monomer, and in which the monomers of the homodimer independently form an internal disulfide bond between the cysteines at positions 35 and 95 and an internal disulfide bond between the cysteines at positions 141 and 199, without being limited thereto.

Meanwhile, as used herein, the term "combination" refers to formation of a linkage between a polypeptide encoding a single-chain immunoglobulin Fc region of the same origin and a single-chain polypeptide of a different origin when a dimer or a multimer is formed. That is, a dimer or multimer may be prepared using two or more Fc fragments selected from the group consisting of IgG Fc, IgA Fc, IgM Fc, IgD Fc, and IgE Fc fragments.

In addition, the above-described conjugate may exhibit an extended duration of effects compared to the native GLP-1, GIP, or glucagon, or compared to X not modified with F, and the conjugate may also include a form enclosed in biodegradable nanoparticles as well as those described above.

The composition including the peptide (e.g., the peptide alone or in a form linked to the biocompatible material) may be used to prevent or treat hyperlipidemia.

As used herein, the term "prevention" refers to all actions intended to inhibit or delay development of hyperlipidemia by administering the peptide or the composition including the same. The term "treatment" refers to all actions to alleviate or beneficially change symptoms associated with hyperlipidemia by administering the peptide or the composition including the same.

As used herein, the "administration" refers to introduction of a particular substance into a patient by any appropriate method, and an administration route of the composition may be, but is not limited to, any conventional route that enables delivery of the composition to the target in living organisms, for example, intraperitoneal administration, intravenous administration, intramuscular administration, subcutaneous administration, intradermal administration, oral administration, topical administration, intranasal administration, intrapulmonary administration, or intrarectal administration.

The pharmaceutical composition of the present invention may further include a pharmaceutically acceptable carrier, excipient, or diluent. The pharmaceutically acceptable carrier, excipient, or diluent may be one which is not naturally occurring.

As used herein, the term "pharmaceutically acceptable" refers to an amount sufficient for exhibiting therapeutic effects without causing side effects and may be easily determined by those of ordinary skill in the art based on factors well known in the medical field such as the type of disease, age, body weight, health status, gender, and sensitivity to drug of a patient, administration route, administration method, frequency of administration, duration of treatment, and a drug used in combination or concurrently.

The pharmaceutical composition including the peptide or the conjugate of the present invention may further include a pharmaceutically acceptable carrier. Although the pharmaceutically acceptable carrier is not particularly limited, a binder, a lubricant, a disintegrator, an excipient, a solubilizer, a dispersant, a stabilizer, a suspending agent, a coloring agent, and a flavoring agent may be used for oral administration, a buffer, a preservative, an analgesic, a solubilizer, an isotonic agent, and a stabilizer may be used in combination for injectable preparations, and a base, an excipient, a lubricant, a preservative, and the like may be used for preparation for topical administration.

The composition of the present invention may be formulated into various forms in combination with the above-mentioned pharmaceutically acceptable carrier. For example, for oral administration, the pharmaceutical composition may be formulated into tablets, troches, capsules, elixirs, suspensions, syrups, wafers, and the like. For injectable preparations, the pharmaceutical composition may be formulated into a single-dose ampoule or a multi-dose form. The pharmaceutical composition may also be formulated into solutions, suspensions, tablets, pills, capsules, sustained-release preparations, and the like.

Meanwhile, examples of the carrier, excipient, and diluent suitable for formulation may include lactose, dextrose, sucrose, sorbitol, mannitol, xylitol, erythritol, maltitol, starch, Acacia rubber, alginate, gelatin, calcium phosphate, calcium silicate, cellulose, methyl cellulose, amorphous cellulose, polyvinyl pyrrolidone, water, methylhydroxy benzoate, propylhydroxy benzoate, talc, magnesium stearate, or mineral oils. Also, the pharmaceutical composition may further include a filler, an anti-coagulant, a lubricant, a humectant, a flavoring agent, a preservative, and the like.

In addition, the pharmaceutical composition of the present invention may be formulated in a formulation selected from the group consisting of tablets, pills, powders, granules, capsules, suspensions, formulations for internal use, emulsions, syrups, sterilized aqueous solutions, non-aqueous solvents, lyophilized preparations, and suppositories.

Also, the composition may be formulated in a unit dosage form suitable for administration into the body of a patient, specifically in a form useful for administration of protein medicines, according to a method commonly used in the art and administered via an oral administration route or a parenteral administration route such as an intradermal, intravenous, intramuscular, intraarterial, intramedullary, intrathecal, intraventricular, intrapulmonary, transdermal, subcutaneous, intraperitoneal, intranasal, intestinal, topical, sublingual, vaginal, or rectal route using an administration method commonly used in the art, but is not limited thereto.

In addition, the peptide or the conjugate thereof may be used in combination with various carriers permitted as medicaments such as a saline solution or an organic solvent. As the medicaments, carbohydrates such as glucose, sucrose, or dextran, antioxidants such as ascorbic acid or glutathione, chelating agents, low-molecular-weight proteins, or other stabilizers may be used to improve stability or absorbability.

An administration dose and frequency of the pharmaceutical composition of the present invention may be determined depending on type of a drug, as an active ingredient, together with various related factors such as disease to be treated, administration route, age, gender, and body weight of a patient, and severity of disease.

A total effective amount of the composition of the present invention may be administered to a patient in a single dose or in multiple doses using a fractional treatment protocol in which administration is performed for a prolonged period of time. The amount of an active ingredient of the pharmaceutical composition of the present invention may vary according to severity of a disease. Specifically, a preferred daily dosage of the conjugate of the present invention may be from about 0.0001 mg to about 500 mg per 1 kg of the body weight of the patient. However, since the dosage of the conjugate is determined as an effective dosage for the patient in consideration of various factors such as age, body weight, health status, and gender of the patient, severity of disease, diet, and excretion rate as well as route and frequency of administration of the pharmaceutical composition, an appropriate effective dosage for a particular use of the composition of the present invention may be determined by one or ordinary skill in the art by considering these factors. The formulation, administration route, and administration method of the pharmaceutical composition are not particularly limited as long as the effects of the present invention are obtained.

Since the pharmaceutical composition of the present invention has excellent *in vivo* persistence and potency, the number and frequency of administration of the pharmaceutical composition according to the present invention may be significantly reduced, without being limited thereto.

Another aspect of the present invention provides a method of preventing or treating hyperlipidemia, the method including administering the peptide, the conjugate thereof, or the composition including the same to an individual in need thereof.

The peptide, the conjugate thereof, or the composition including the same, hyperlipidemia, prevention, and treatment are as described above.

In the present invention, the individual refers to a subject suspected to have hyperlipidemia, and the subject suspected to have hyperlipidemia refers to mammals such as rats and livestock including humans with the disease or at risk of developing the disease, but any individual that may be treated with the conjugate or the composition including the same according to the present invention may be included without limitation.

The method of the present invention may include administering the pharmaceutical composition including the peptide or the conjugate thereof in a pharmaceutically effective amount. An appropriate daily dose may be determined by a doctor within the scope of sound medical judgment in a bolus or in multiple doses. However, for the purpose of the present invention, it is preferred that a specific therapeutically effective amount for a particular patient is differently applied depending on various factors including the type and extent of a response to be achieved, a specific composition including whether other formulations are used according to the case, age, body weight, general health status, gender, and diet of the patient, administration time, administration route, excretion rate of the composition, duration of treatment, a drug used in combination or concurrently with the specific composition, and similar factors well known in the medical field.

Another aspect of the present invention provides a use of the peptide, the conjugate thereof, or the composition including the same for preparation of medicaments for preventing or treating hyperlipidemia.

The peptide, the conjugate thereof, or the composition including the same, hyperlipidemia, prevention, and treatment are as described above.

Hereinafter, the present invention will be described in more detail with reference to the following examples. However, the following examples are merely presented to exemplify the present invention, and the scope of the present invention is not limited thereto.

### Example 1: Preparation of Triple Agonist

Triple agonists having activity to all of the GLP-1, GIP, and glucagon receptors were prepared, and sequences thereof are listed in Table 2 below.

**Table 2**

| **SEQ ID NO:** | **Sequence** | **Informati on** |
|---|---|---|
| 1 | H X Q G T F T S D V S S Y L D G Q A A K E F I A W L V K G C | |
| 2 | H X Q G T F T S D V S S Y L D G Q A Q K E F I A W L V K G C | |
| 3 | H X Q G T F T S D V S S Y L L G Q A A K Q F I A W L V K G G G P S S G A P P P S C | |
| 4 | H X Q G T F T S D V S S Y L L G Q Q Q K E F I A W L V K G C | |
| 5 | H X Q G T F T S D V S S Y L L G Q Q Q K E F I A W L V K G G G P S S G A P P P S C | |
| 6 | H X Q G T F T S D V S S Y L D G Q A A K E F V A W L L K G C | |
| 7 | H X Q G T F T S D V S K Y L D G Q A A K E F V A W L L K G C | |
| 8 | H X Q G T F T S D V S K Y L D G Q A A Q E F V A W L L K G C | |
| 9 | H X Q G T F T S D V S K Y L D G Q A A Q E F V A W L L A G C | |
| 10 | H X Q G T F T S D V S K Y L D G Q A A Q E F V A W L L A G G G P S S G A P P P S C | |
| 11 | CAGEGTFTSDLSKYLDSRRQQLFVQWLK AGGPSSGAPPPSHG | |
| 12 | CAGEGTFISDLSKYMDEQAVQLFVEWLM AGGPSSGAPPPSHG | |
| 13 | CAGEGTFISDYSIQLDEIAVQDFVEWLLA QKPSSGAPPPSHG | |
| 14 | CA G Q G T F T S D Y S I Q L D E I A V R D F V E W L K N G G P S S G A P P P S H G | |
| 15 | CAGQGTFTSDLSKQMDEEAVRLFIEWLK NGGPSSGAPPPSHG | |
| 16 | CAGQGTFTSDLSKQMDSEAQQLFIEWLK NGGPSSGAPPPSHG | |
| 17 | CAGQGTFTSDLSKQMDEERAREFIEWLL AQKPSSGAPPPSHG | |
| 18 | CAGQGTFTSDLSKQMDSERAREFIEWLK NTGPSSGAPPPSHG | |
| 19 | CA G Q G T F T S D L S I Q Y D S E H Q R D F I E W L K D T G P S S G A P P P S H G | |
| 20 | CAGQGTFTSDLSIQYEEEAQQDFVEWLK DTGPSSGAPPPSHG | |
| 21 | Y X Q G T F T S D Y S K Y L D E C R A K E F V Q W L L D H H P S S G Q P P P S | Ring formed |
| 22 | Y X Q G T F T S D Y S K C L D E K R A K E F V Q W L L D H H P S S G Q P P P S | Ring formed |
| 23 | Y X Q G T F T S D Y S K Y L D E C R A K E F V Q W L L A Q K G K K N D W K H N I T | Ring formed |
| 24 | Y X Q G T F T S D Y S K Y L D E C R A K E F V Q W L K N G G P S S G A P P P S | Ring formed |
| 25 | H X Q G T F T S D C S K Y L D E R A A Q D F V Q W L L D G G P S S G A P P P S | |
| 26 | H X Q G T F T S D C S K Y L D S R A A Q D F V Q W L L D G G P S S G A P P P S | |
| 27 | H X Q G T F T S D Y S K Y L D E R A C Q D F V Q W L L D Q G G P S S G A P P P S | |
| 28 | H X Q G T F T S D Y S K Y L D E K R A Q E F V C W L L A Q K G K K N D W K H N I T | |
| 29 | H X Q G T F T S D Y S K Y L D E K A A K E F V Q W L L N T C | Ring formed |
| 30 | H X Q G T F T S D Y S K Y L D E K A Q K E F V Q W L L D T C | Ring formed |
| 31 | H X Q G T F T S D Y S K Y L D E K A C K E F V Q W L L A Q | Ring formed |
| 32 | H X Q G T F T S D Y S K Y L D E K A C K D F V Q W L L D G G P S S G A P P P S | Ring formed |
| 33 | H X Q G T F T S D Y S I A M D E I H Q K D F V N W L L A Q K C | Ring formed |
| 34 | H X Q G T F T S D Y S K Y L D E K R Q K E F V N W L L A Q K C | Ring formed |
| 35 | H X Q G T F T S D Y S I A M D E I H Q K D F V N W L L N T K C | Ring formed |
| 36 | H X Q G T F T S D Y S K Y L C E K R Q K E F V Q W L L N G G P S S G A P P P S G | Ring formed |
| 37 | H X Q G T F T S D Y S K Y L D E C R Q K E F V Q W L L N G G P S S G A P P P S G | Ring formed |
| 38 | CA X Q G T F T S D K S S Y L D E R A A Q D F V Q W L L D G G P S S G A P P P S S | |
| 39 | H X Q G T F T S D Y S K Y L D G Q H A Q C F V A W L L A G G G P S S G A P P P S | |
| 40 | H X Q G T F T S D K S K Y L D E R A C Q D F V Q W L L D G G P S S G A P P P S | |
| 41 | H X Q G T F T S D K S K Y L D E C A A Q D F V Q W L L D G G P S S G A P P P S | |
| 42 | Y X Q G T F T S D Y S K Y L D E K R A K E F V Q W L L D H H P S S G Q P P P S C | Ring formed |
| 43 | Y X Q G T F T S D Y S K Y L D E K R A K E F V Q W L L D H H C S S G Q P P P S | Ring formed |
| 44 | H G Q G T F T S D C S K Q L D G Q A A Q E F V A W L L A G G P S S G A P P P S | |
| 45 | H G Q G T F T S D C S K Y M D G Q A A Q D F V A W L L A G G P S S G A P P P S | |
| 46 | H G Q G T F T S D C S K Y L D E Q H A Q E F V A W L L A G G P S S G A P P P S | |
| 47 | HGQGTFTSDCSKYLDGQRAQEFVAWLL AGGPSSGAPPPS | |
| 48 | H G Q G T F T S D C S K Y L D G Q R A Q D F V N W L L A G G P S S G A P P P S | |
| 49 | CA X Q G T F T S D Y S I C M D E I H Q K D F V N W L L N T K | Ring formed |
| 50 | H X Q G T F T S D Y S K Y L D E K R A K E F V Q W L L D H H P S S G Q P P P S C | Ring formed |
| 51 | H X Q G T F T S D Y S K Y L D E K R Q K E F V Q W L L N T C | Ring formed |
| 52 | H X Q G T F T S D Y S K Y L D E K R Q K E F V Q W L L D T C | Ring formed |
| 53 | H X E G T F T S D Y S I A M D E I H Q K D F V N W L L A Q C | Ring formed |
| 54 | H X E G T F T S D Y S I A M D E I H Q K D F V D W L L A E C | Ring formed |
| 55 | H X Q G T F T S D Y S I A M D E I H Q K D F V N W L L A Q C | Ring formed |
| 56 | H X Q G T F T S D Y S K Y L D E K R Q K E F V N W L L [?] A Q C | Ring formed |
| 57 | H X Q G T F T S D Y S I A M D E I H Q K D F V N W L L N T C | Ring formed |
| 58 | H X Q G T F T S D Y S K Y L D E K R Q K E F V Q W L L N T K C | Ring formed |
| 59 | CA X Q G T F T S D Y S I C M D E K H Q K D F V N W L L N T K | Ring formed |
| 60 | CA X Q G T F T S D Y S I A M D E K H C K D F V N W L L N T K | Ring formed |
| 61 | CA X Q G T F T S D Y S I A M D E I A C K D F V N W L L N T K | Ring formed |
| 62 | CAXQGTFTSDKSKYLDERAAQDFVQWLL DGGPSSGAPPPS | |
| 63 | CAXQGTFTSDCSKYLDERAAQDFVQWLL DGGPSSGAPPPS | |
| 64 | Y X Q G T F T S D Y S K Y L D E C A A K E F V Q W L L D H H P S S G Q P P P S | Ring formed |
| 65 | H X Q G T F T S D Y S K C L D E K R A K E F V Q W L L D H H P S S G Q P P P S | Ring formed |
| 66 | Y X Q G T F T S D Y S K Y L D E C R A K D F V Q W L L D H H P S S G Q P P P S | Ring formed |
| 67 | Y X Q G T F T S D Y S K Y L D E C A A K D F V Q W L L D H H P S S G Q P P P S | Ring formed |
| 68 | Y X Q G T F T S D Y S K C L D E K A A K E F V Q W L L D H H P S S G Q P P P S | Ring formed |
| 69 | Y X Q G T F T S D Y S K C L D E R A A K E F V Q W L L D H H P S S G Q P P P S | Ring formed |
| 70 | Y X Q G T F T S D Y S K C L D E K R A K D F V Q W L L D H H P S S G Q P P P S | Ring formed |
| 71 | Y X Q G T F T S D Y S K Y L D E R A C K D F V Q W L L D H H P S S G Q P P P S | Ring formed |
| 72 | Y X Q G T F T S D C S K Y L D E R A A K D F V Q W L L D H H P S S G Q P P P S | Ring formed |
| 73 | CAXQGTFTSDYSKYLDECRAKEFVQWLL DHHPSSGQPPPS | Ring formed |
| 74 | CAXQGTFTSDYSKCLDEKRAKEFVQWLL DHHPSSGQPPPS | Ring formed |
| 75 | Y X Q G T F T S D Y S K Y L D E K A A K E F V Q W L L D H H P S S G Q P P P S C | Ring formed |
| 76 | Y X Q G T F T S D Y S K Y L D E K R A K D F V Q W L L D H H P S S G Q P P P S C | Ring formed |
| 77 | Y X Q G T F T S D Y S K Y L D E K A A K D F V Q W L L D H H P S S G Q P P P S C | Ring formed |
| 78 | H X Q G T F T S D Y S K Y L D E K R Q K E F V Q W L L D T K C | Ring formed |
| 79 | H X E G T F T S D Y S I A M D E I H Q K D F V N W L L A Q K C | Ring formed |
| 80 | H X E G T F T S D Y S I A M D E I H Q K D F V D W L L A E K C | Ring formed |
| 81 | CA X Q G T F T S D Y S K Y L D E K R Q K E F V Q W L L N T C | Ring formed |
| 82 | CAXQGTFTSDYSKYLDEKRQKEFVQWLL DTC | Ring formed |
| 83 | CA X E G T F T S D Y S I A M D E I H Q K D F V N W L L A Q C | Ring formed |
| 84 | CA X E G T F T S D Y S I A M D E I H Q K D F V D W L L A E C | Ring formed |
| 85 | CA X Q G T F T S D Y S I A M D E I H Q K D F V N W L L A Q C | Ring formed |
| 86 | CAXQGTFTSDYSKYLDEKRQKEFVNWLL AQC | Ring formed |
| 87 | CA X Q G T F T S D Y S I A M D E I H Q K D F V N W L L N T C | Ring formed |
| 88 | CAXQGTFTSDYSKYLDEKRQKEFVQWLL NTKC | Ring formed |
| 89 | CA X Q G T F T S D Y S K Y L D E K R Q K E F V Q W L L D T K C | Ring formed |
| 90 | CAXEGTFTSDYSIAMDEIHQKDFVNWLL AQKC | Ring formed |
| 91 | CA X E G T F T S D Y S I A M D E I H Q K D F V D W L L A E K C | Ring formed |
| 92 | CA X Q G T F T S D Y S I A M D E I H Q K D F V N W L L A Q K C | Ring formed |
| 93 | CA X Q G T F T S D Y S K Y L D E K R Q K E F V N W L L A Q K C | Ring formed |
| 94 | CA X Q G T F T S D Y S I A M D E I H Q K D F V N W L L N T K C | Ring formed |
| 95 | Y X Q G T F T S D Y S K Y L D E K R A K E F V Q W L L C H H P S S G Q P P P S | Ring formed |
| 96 | Y X Q G T F T S D Y S K Y L D E K R A K E F V Q W L L D H C P S S G Q P P P S | Ring formed |
| 97 | Y X Q G T F T S D Y S K Y L D E K R A K E F V Q W L L D C H P S S G Q P P P S | Ring formed |
| 98 | Y X Q G T F T S D Y S K A L D E K A A K E F V N W L L D H H P S S G Q P P P S C | Ring formed |
| 99 | Y X Q G T F T S D Y S K A L D E K A A K D F V N W L L D H H P S S G Q P P P S C | Ring formed |
| 100 | Y X Q G T F T S D Y S K A L D E K A A K E F V Q W L L D Q H P S S G Q P P P S C | Ring formed |
| 101 | Y X Q G T F T S D Y S K A L D E K A A K E F V N W L L D Q H P S S G Q P P P S C | Ring formed |
| 102 | Y X Q G T F T S D Y S K A L D E K A A K D F V N W L L D Q H P S S G Q P P P S C | Ring formed |

In the sequences shown in Table 2, the amino acid marked with X represents a non-native amino acid, aminoisobutyric acid (Aib), and the underlined amino acids represent formation of a ring therebetween. Also, in Table 2, CA is 4-imidazoacetyl, and Y is tyrosine.

### Example 2: Preparation of long-acting conjugate of triple agonist

For pegylation of 10 kDa PEG having a maleimide group and an aldehyde group at both ends respectively, i.e., maleimide-PEG-aldehyde (10 kDa, NOF, Japan), into a cysteine residue of each triple agonist of Example 1 (SEQ ID NOS: 21, 22, 42, 43, 50, 77, and 96), the triple agonist and the maleimide-PEG-aldehyde were reacted at a molar ratio of 1:1 to 3 with a protein concentration of 1 mg/mL to 5 mg/mL at low temperature for 0.5 to 3 hours. In this case, the reaction was conducted in an environment including 50 mM Tris buffer (pH 7.5) to which 20% to 60% isopropanol was added. Upon completion of the reaction, the reaction solution was applied to SP sepharose HP (GE Healthcare, USA) to purify the triple agonist mono-pegylated on cysteine.

Subsequently, the purified mono-pegylated triple agonist and an immunoglobulin Fc were reacted at a molar ratio of 1:1 to 5 with a protein concentration of 10 mg/mL to 50 mg/mL at a temperature of 4°C to 8°C for 12 to 18 hours. The reaction was conducted in an environment in which 10 mM to 50 mM sodium cyanoborohydride, as a reducing agent, and 10% to 30% isopropanol were added to a 100 mM calcium phosphate buffer (pH 6.0). Upon completion of the reaction, the reactant solution was applied to a butyl sepharose FF purification column (GE Healthcare, USA) and a Source ISO purification column (GE Healthcare, USA) to purify the conjugate including the triple agonist and the immunoglobulin Fc. The purified long-acting conjugate has a structure in which the triple agonist, polyethylene glycol (PEG) linker, and Fc dimer are covalently linked at a 1:1:1 molar ratio, and the PEG linker is linked to only one chain of the two polypeptide chains of the Fc dimer.

Meanwhile, the immunoglobulin Fc is one in which two monomers having the amino acid sequence of SEQ ID NO: 139 (consisting of 221 amino acids) form a homodimer through a disulfide bond between cysteines, which are the 3^{rd} amino acid of each monomer, and in which the monomers of the homodimer independently form an internal disulfide bond between the cysteines at positions 35 and 95 and an internal disulfide bond between the cysteines at positions 141 and 199.

After preparation, purity analyzed by reverse-phase chromatography, size-exclusion chromatography, and ion-exchange chromatography was 95% or more.

The conjugate in which the triple agonist prepared in the example is linked to the immunoglobulin Fc via PEG was named as a conjugate including the triple agonist and the immunoglobulin Fc or a long-acting conjugate.

For example, the conjugate in which the triple agonist of SEQ ID NO: 42 is linked to the immunoglobulin Fc via PEG was named "conjugate including SEQ ID NO: 42 and immunoglobulin Fc" or "long-acting conjugate of SEQ ID NO: 42", which may be used interchangeably herein.

### Experimental Example 1: Measurement of in vitro activity of triple agonist and long-acting conjugate thereof

Activities of the triple agonists and the long-acting conjugates thereof respectively prepared in Examples 1 and 2 were measured using transformed cell lines in which a GLP-1 receptor, a glucagon (GCG) receptor, and a GIP receptor were transformed by way of a method of measuring *in vitro* cellular activities.

The cell lines were transformed such that genes for the human GLP-1 receptor, the human GCG receptor, and the human GIP receptor were each expressed in Chinese hamster ovary (CHO), and were suitable for measuring activities of GLP-1, GCG, and GIP, respectively. Therefore, the activity for each part was measured using each of the transformed cell lines.

For measurement of the activity of each of the triple agonists and the long-acting conjugates thereof respectively prepared in Examples 1 and 2 to GLP-1, human GLP-1 was subjected to a 4-fold serial dilution from 50 nM to 0.000048 nM, and the triple agonists and the long-acting conjugates thereof respectively prepared in Examples 1 and 2 were subjected to a 4-fold serial dilution from 400 nM to 0.00038 nM. A cultured solution was removed from the cultured CHO cells in which the human GLP-1 receptor was expressed, and each of the serially diluted substances was added to the CHO cells in an amount of 5 µL. Then, a buffer solution including cAMP antibody was added thereto in an amount of 5 µL and cultured at room temperature for 15 minutes. Thereafter, a detection mix including a cell lysis buffer was added thereto in an amount of 10 µL for lysis of the cells, followed by reaction at room temperature for 90 minutes. Upon completion of the reaction, the cell lysates were applied to a LANCE cAMP kit (PerkinElmer, USA) to calculate EC₅₀ values via accumulated cAMP, and the values were compared with each other. Relative potencies compared to human GLP-1 are shown in Tables 3 and 4 below.

For measurement of the activity of the triple agonists and the long-acting conjugates thereof respectively prepared in Examples 1 and 2 to GCG, human GCG was subjected to a 4-fold serial dilution from 50 nM to 0.000048 nM, and the triple agonists and the long-acting conjugates thereof respectively prepared in Examples 1 and 2 were subjected to a 4-fold serial dilution from 400 nM to 0.00038 nM. A cultured solution was removed from the cultured CHO cells in which the human GCG receptor was expressed, and each of the serially diluted substances was added to the CHO cells in an amount of 5 µL. Then, a buffer solution including cAMP antibody was added thereto in an amount of 5 µL and cultured at room temperature for 15 minutes. Thereafter, a detection mix including a cell lysis buffer was added thereto in an amount of 10 µL for lysis of the cells, followed by reaction at room temperature for 90 minutes. Upon completion of the reaction, the cell lysates were applied to a LANCE cAMP kit (PerkinElmer, USA) to calculate EC₅₀ values via accumulated cAMP, and the values were compared with each other. Relative potencies compared to human GCG are shown in Tables 3 and 4 below.

For measurement of the activity of the triple agonists and the long-acting conjugates thereof respectively prepared in Examples 1 and 2 to GIP, human GIP was subjected to a 4-fold serial dilution from 50 nM to 0.000048 nM, and the triple agonists and the long-acting conjugates thereof respectively prepared in Examples 1 and 2 were subjected to a 4-fold serial dilution from 400 nM to 0.00038 nM. A cultured solution was removed from the cultured CHO cells in which the human GIP receptor was expressed, and each of the serially diluted substances was added to the CHO cells in an amount of 5 µL. Then, a buffer solution including cAMP antibody was added thereto in an amount of 5 µL and cultured at room temperature for 15 minutes. Thereafter, a detection mix including a cell lysis buffer was added thereto in an amount of 10 µL for lysis of the cells, followed by reaction at room temperature for 90 minutes. Upon completion of the reaction, the cell lysates were applied to a LANCE cAMP kit (PerkinElmer, USA) to calculate EC₅₀ values via accumulated cAMP, and the values were compared with each other. Relative potencies compared to human GIP are shown in Tables 3 and 4 below.

**Table 3**

| Relative potency ratio of triple agonist | | | |
|---|---|---|---|
| | *In vitro* activity relative to native peptide (%) | | |
| **SEQ ID NO:** | *vs*. GLP-1 | *vs.* Glucagon | *vs*. GIP |
| 1 | 3.2 | <0.1 | <0.1 |
| 2 | 5.9 | <0.1 | <0.1 |
| 3 | 1.8 | <0.1 | <0.1 |
| 4 | 8.5 | <0.1 | <0.1 |
| 5 | 42.1 | <0.1 | <0.1 |
| 6 | 17.0 | <0.1 | <0.1 |
| 7 | 13.7 | <0.1 | <0.1 |
| 8 | 14.2 | 0.10 | <0.1 |
| 9 | 32.1 | 0.13 | <0.1 |
| 10 | 46.0 | <0.1 | <0.1 |
| 11 | 1.4 | <0.1 | <0.1 |
| 12 | 0.4 | <0.1 | <0.1 |
| 13 | <0.1 | <0.1 | <0.1 |
| 14 | 28.0 | <0.1 | <0.1 |
| 15 | 79.2 | <0.1 | <0.1 |
| 16 | 2.1 | <0.1 | <0.1 |
| 17 | 0.2 | <0.1 | <0.1 |
| 18 | <0.1 | <0.1 | <0.1 |
| 19 | <0.1 | <0.1 | <0.1 |
| 20 | <0.1 | <0.1 | <0.1 |
| 21 | 17.8 | 267 | 22.7 |
| 22 | 20.1 | 140 | 59.7 |
| 23 | 4.01 | 9.3 | <0.1 |
| 24 | 41.2 | 9.3 | <0.1 |
| 25 | 82.6 | 0.1 | <0.1 |
| 26 | 64.5 | 0.2 | <0.1 |
| 27 | 83.1 | 0.8 | 0.9 |
| 28 | 17.2 | 1.6 | <0.1 |
| 29 | 38.5 | 6.0 | <0.1 |
| 30 | 142 | 0.7 | 0.8 |
| 31 | 135 | 2.2 | 2.4 |
| 32 | 151 | 1.7 | 8.8 |
| 33 | 24.5 | <0.1 | 10.4 |
| 34 | 19.1 | 0.92 | 0.6 |
| 35 | 7.5 | <0.1 | 1.3 |
| 36 | 37.4 | 0.39 | 0.2 |
| 37 | 236 | 6.21 | 2.2 |
| 38 | 2.3 | - | - |
| 39 | 13.9 | 0.53 | <0.1 |
| 40 | 75.2 | <0.1 | <0.1 |
| 41 | 34.3 | <0.1 | <0.1 |
| 42 | 33.9 | 205.8 | 7.8 |
| 43 | 12.6 | 88.4 | 3.70 |
| 44 | 1.3 | <0.1 | <0.1 |
| 45 | 6.6 | <0.1 | <0.1 |
| 46 | 1.4 | <0.1 | <0.1 |
| 47 | 2.4 | <0.1 | <0.1 |
| 48 | 1.5 | <0.1 | <0.1 |
| 49 | 29.8 | <0.1 | 3.3 |
| 50 | 67.4 | 50.5 | 2.7 |
| 51 | 14.4 | 2.0 | 0.1 |
| 52 | 44.1 | 7.5 | 0.3 |
| 53 | 161 | 8.4 | 1.3 |
| 54 | 30.6 | 1.4 | 0.1 |
| 55 | 27.1 | 0.7 | 2.4 |
| 56 | 57.9 | 4.9 | 0.8 |
| 57 | 11.7 | <0.1 | 0.3 |
| 58 | 39.1 | 2.6 | 0.2 |
| 59 | 40.3 | <0.1 | 4.0 |
| 60 | 106.2 | <0.1 | 8.2 |
| 61 | 59.8 | <0.1 | 2.8 |
| 62 | 5.2 | <0.1 | <0.1 |
| 63 | 15.3 | <0.1 | <0.1 |
| 64 | 64.6 | 60.1 | 92.9 |
| 65 | 95.4 | 25.2 | 11.6 |
| 66 | 15.8 | 172 | 17.2 |
| 67 | 28.5 | 46.2 | 39.8 |
| 68 | 27.9 | 8.8 | 107 |
| 69 | 24.3 | 9.6 | 62.8 |
| 70 | 15.1 | 71.3 | 64.4 |
| 71 | 90.1 | 12.7 | 94.7 |
| 72 | 11.5 | 1.0 | 1.6 |
| 73 | 22.6 | 5.4 | 3.0 |
| 74 | 12.9 | 0.9 | 1.0 |
| 75 | 35.1 | 8.5 | 18.0 |
| 76 | 10.3 | 47.6 | 11.7 |
| 77 | 38.7 | 12.2 | 35.5 |
| 78 | 51.0 | 14.0 | 0.12 |
| 79 | 41.5 | 4.9 | 1.4 |
| 80 | 8.1 | 0.0 | 0.1 |
| 81 | 7.8 | 0.3 | <0.1 |
| 82 | 9.5 | 1.1 | <0.1 |
| 83 | 47.3 | 1.3 | 0.4 |
| 84 | 4.2 | <0.1 | <0.1 |
| 85 | 4.3 | <0.1 | 0.3 |
| 86 | 28.4 | 0.4 | 0.2 |
| 87 | 0.9 | <0.1 | <0.1 |
| 88 | 9.6 | 0.3 | <0.1 |
| 89 | 7.1 | 0.7 | <0.1 |
| 90 | 7.4 | <0.1 | <0.1 |
| 91 | 31.9 | 16.8 | 0.3 |
| 92 | 0.8 | <0.1 | 0.4 |
| 93 | 5.7 | 0.3 | 0.7 |
| 94 | 0.5 | <0.1 | <0.1 |
| 95 | 2.1 | 0.4 | <0.1 |
| 96 | 34.4 | 194.8 | 5.2 |
| 97 | 10.5 | 62.8 | 2.6 |
| 98 | 28.1 | 8.2 | 47.1 |
| 99 | 20.9 | 14.9 | 57.7 |
| 100 | 42.2 | 12.7 | 118.5 |
| 101 | 23.2 | 13.9 | 40.1 |
| 102 | 23.3 | 29.5 | 58.0 |

**Table 4**

| Long-acting conjugate | *In vitro* activity relative to native peptide (%) | | |
|---|---|---|---|
| | *vs*. GLP-1 | *vs*. Glucagon | *vs*. GIP |
| 21 | 0.1 | 1.6 | 0.2 |
| 22 | 0.1 | 0.9 | 0.5 |
| 42 | 3.1 | 23.1 | 1.2 |
| 43 | 2.1 | 13.5 | 0.6 |
| 50 | 15.4 | 6.9 | 0.7 |
| 77 | 6.7 | 1.7 | 6.6 |
| 96 | 0.3 | 4.0 | 0.3 |

Table 4 shows relative potency ratios of the triple agonists and the long-acting conjugates thereof, and it was confirmed that the novel long-acting conjugates of the triple agonists prepared as described above have functions of activating all of the GLP-1 receptor, the GIP receptor, and the glucagon receptor.

### Experimental Example 2: Identification of therapeutic effect of long-acting conjugate of triple agonist on hyperlipidemia (in vivo)

Golden Syrian hamsters (hereinafter referred to as hyperlipidemia hamsters) were fed with fructose for 2 weeks to induce a hyperlipidemia model. Among the triple agonists prepared in Example 1, SEQ ID NO: 42 (1.6 nmol/kg, 3.1 nmol/kg, Q2D) was selected, and the long-acting conjugate of the triple agonist having SEQ ID NO: 42 was repeatedly administered to the hamsters induced with hyperlipidemia for 3 weeks by subcutaneous administration. As a comparative example, therapeutic effects of evolocumab (219.2 nmol/kg, QW), as a conventional therapeutic agent, on hyperlipidemia were measured.

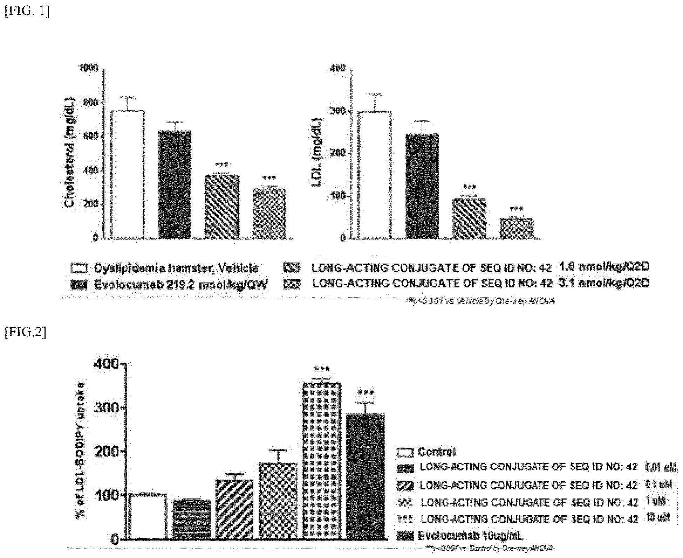

As shown in FIG. 1, it was confirmed that the total blood cholesterol level (decreased by 50% to 60%) and the blood LDL level (decreased by 70% to 85%) were significantly decreased by repeatedly administering the long-acting conjugate of SEQ ID NO: 42 for 3 weeks, while negligible effects were observed in the group administered with evolocumab.

### Experimental Example 3: Identification of therapeutic effect of long-acting conjugate of triple agonist on hyperlipidemia (in vitro)

Through experiments for identifying effects on increasing LDL absorption, inhibiting the activity of 3-hydroxy-3-methylglutaryl-CoA reductase (HMGCR), and promoting fatty acid degradation using HepG2 cells, it was confirmed that excellent in *vitro* therapeutic mode of action of the long-acting conjugate of the triple agonist of Example 2 on hyperlipidemia were confirmed.

### (1) Increase in LDL absorption by long-acting conjugate of triple agonist

Compared to the control in which HepG2 cells were not treated with a long-acting conjugate of SEQ ID NO: 42, a long-acting conjugate of SEQ ID NO: 42 (0.01 µM, 0.1 µM, 1 µM, and 10 µM), and evolocumab (10 µg/mL) for 48 hours, amounts of absorbed BODIPY-labeled LDL were measured in the cells, and the results are shown in FIG. 2.

As shown in FIG. 2, it was confirmed that the amounts of absorbed LDL increased by the long-acting conjugate of SEQ ID NO: 42 in a concentration-dependent manner (increased by 132.7% to 354% relative to the control).

### (2) Inhibition of activity of 3-hydroxy-3-methylglutaryl-CoA reductase (HMGCR) by long-acting conjugate of triple agonist

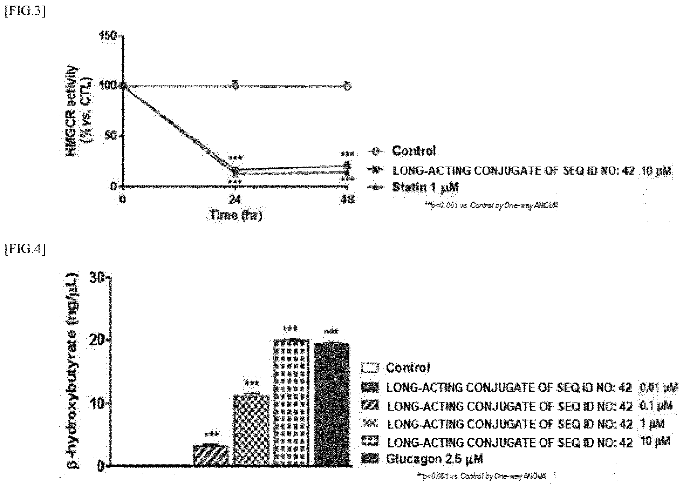

In order to measure the activity of 3-hydroxy-3-methylglutaryl-CoA reductase (hereinafter referred to as HMGCR), which is a rate-limiting enzyme of cholesterol synthesis, compared to the control in which HepG2 cells were not treated with a long-acting conjugate of SEQ ID NO: 42, HepG2 cells were treated with the long-acting conjugate of SEQ ID NO: 42(10 µM) and statin (1 µM), and then the degree of NADPH reduction was measured under the treatment conditions with HMG-CoA (HMGCR converts HMG-COA into a cholesterol precursor, mevalonate, by using NADPH), and the results are shown in FIG. 3.

As shown in FIG. 3, it was confirmed that the long-acting conjugate of SEQ ID NO: 42 continuously inhibited the activity of HMGCR (decrease by about 80% relative to the control).

### (3) Promoting degradation of fatty acid by long-acting conjugate of triple agonist

Ketone bodies, which are a by-product of fatty acid degradation, were measured. To this end, compared to the control in which HepG2 cells were not treated with a long-acting conjugate of SEQ ID NO: 42, the HepG2 cells treated with a fatty acid (palmitate) were treated with the long-acting conjugate of SEQ ID NO: 42, (0.01 µM, 0.1 µM, 1 µM, and 10 µM) and glucagon (2.5 µM), and then the amount of generated ketone bodies was measured using a commercially available ketone body measuring kit (Sigma #MAK041).

As a result of measurement, as shown in FIG. 4, it was confirmed that the ketone body generation was increased by the long-acting conjugate of SEQ ID NO: 42 in a concentration-dependent manner.

Based on the above-described experiment, it was confirmed that the triple agonist and the long-acting conjugate thereof according to the present invention have therapeutic effects on hyperlipidemia by increasing LDL absorption, inhibiting cholesterol synthesis by inhibiting the activity of HMGCR, and reducing the triglyceride level and the LDL level by promoting degradation of fatty acids.

The above description of the present invention is provided for the purpose of illustration, and it would be understood by those skilled in the art that various changes and modifications may be made swithout changing the technical conception and essential features of the present invention. Thus, it is clear that the above-described embodiments are illustrative in all aspects and do not limit the present invention. The various embodiments disclosed herein are not intended to be limiting, with the true scope and spirit being indicated by the following claims. The present invention is to be limited only by the terms of the appended claims, along with the full scope of equivalents to which such claims are entitled.

## Claims

1. A pharmaceutical composition for preventing or treating hyperlipidemia comprising an isolated peptide with activity to a glucagon receptor, a glucagon-like peptide-1 (GLP-1) receptor, and a glucose-dependent insulinotropic polypeptide (GIP) receptor,
wherein the peptide comprises an amino acid sequence represented by General Formula 1 below:
Xaa1-Xaa2-Xaa3-Gly-Thr-Phe-Xaa7-Ser-Asp-Xaa10-Ser-Xaa12-Xaa13 -Xaa 14-Xaa 15-Xaa 16-Xaa 17-Xaa 18-Xaa 19-Xaa20-Xaa21 -Phe-Xaa23-Xaa24-Trp-Leu-Xaa27-Xaa28-Xaa29-Xaa30-R1 (General Formula 1, SEQ ID NO: 103)
wherein in General Formula 1 above, Xaa1 is histidine (His, H), 4-imidazoacetyl (CA), or tyrosine (Tyr, Y), Xaa2 is glycine (Gly, G), α-methyl-glutamic acid, or aminoisobutyric acid (Aib), Xaa3 is glutamic acid (Glu, E) or glutamine (Gln, Q), Xaa7 is threonine (Thr, T) or isoleucine (Ile, I), Xaa10 is leucine (Leu, L), tyrosine (Tyr, Y), lysine (Lys, K), cysteine (Cys, C), or valine (Val, V), Xaa12 is lysine (Lys, K), serine (Ser, S), or isoleucine (Ile, I), Xaa13 is glutamine (Gln, Q), tyrosine (Tyr, Y), alanine (Ala, A), or cysteine (Cys, C), Xaa14 is leucine (Leu, L), methionine (Met, M), or tyrosine (Tyr, Y), Xaa15 is cysteine (Cys, C), aspartic acid (Asp, D), glutamic acid (Glu, E), or leucine (Leu, L), Xaa16 is glycine (Gly, G), glutamic acid (Glu, E), or serine (Ser, S), Xaa17 is glutamine (Gln, Q), arginine (Arg, R), isoleucine (Ile, I), glutamic acid (Glu, E), cysteine (Cys, C), or lysine (Lys, K), Xaa18 is alanine (Ala, A), glutamine (Gln, Q), arginine (Arg, R), or histidine (His, H), Xaa19 is alanine (Ala, A), glutamine (Gln, Q), cysteine (Cys, C), or valine (Val, V), Xaa20 is lysine (Lys, K), glutamine (Gln, Q), or arginine (Arg, R), Xaa21 is glutamic acid (Glu, E), glutamine (Gln, Q), leucine (Leu, L), cysteine (Cys, C), or aspartic acid (Asp, D), Xaa23 is isoleucine (Ile, I) or valine (Val, V), Xaa24 is alanine (Ala, A), glutamine (Gln, Q), cysteine (Cys, C), asparagine (Asn, N), aspartic acid (Asp, D), or glutamic acid (Glu, E), Xaa27 is valine (Val, V), leucine (Leu, L), lysine (Lys, K), or methionine (Met, M), Xaa28 is cysteine (Cys, C), lysine (Lys, K), alanine (Ala, A), asparagine (Asn, N), or aspartic acid (Asp, D), Xaa29 is cysteine (Cys, C), glycine (Gly, G), glutamine (Gln, Q), threonine (Thr, T), glutamic acid (Glu, E), or histidine (His, H), Xaa30 is cysteine (Cys, C), glycine (Gly, G), lysine (Lys, K), or histidine (His, H), or is absent, and
R1 is cysteine (Cys, C), GKKNDWKHNIT (SEQ ID NO: 106), m-SSGAPPPS-n (SEQ ID NO: 107), or m-SSGQPPPS-n (SEQ ID NO: 108), or is absent,
wherein m is -Cys-, -Pro-, or -Gly-Pro-, and n is -Cys-, -Gly-, -Ser-, or -His-Gly-, or is absent.

2. The pharmaceutical composition of claim 1, wherein in General Formula 1 above, Xaa14 is leucine or methionine, and Xaa15 is cysteine, aspartic acid, or leucine.

3. The pharmaceutical composition of claim 1, wherein in General Formula 1 above, Xaa2 is glycine, α-methyl-glutamic acid, or Aib, Xaa7 is threonine, Xaa10 is tyrosine, cysteine, or valine, Xaa12 is lysine or isoleucine, Xaa13 is tyrosine, alanine, glutamine, or cysteine, Xaa14 is leucine, cysteine, or methionine, Xaa15 is cysteine, leucine, glutamic acid, or aspartic acid, Xaa17 is glutamine, arginine, isoleucine, cysteine, glutamic acid, or lysine, Xaa18 is alanine, glutamine, arginine, or histidine, Xaa19 is alanine, glutamine, valine, or cysteine, Xaa20 is lysine, arginine, or glutamine, Xaa21 is glutamic acid, glutamine, leucine, cysteine, or aspartic acid, Xaa23 is isoleucine or valine, Xaa24 is cysteine, alanine, glutamine, asparagine, glutamic acid, or aspartic acid, and Xaa27 is leucine or lysine.

4. The pharmaceutical composition of claim 1, wherein the peptide is a peptide comprising an amino acid sequence of General Formula 2 below:
Xaa1-Xaa2-Gln-Gly-Thr-Phe-Thr-Ser-Asp-Xaa10-Ser-Lys-Xaa13-Xaa1 4-Xaa15-Xaa16-Xaa17-Xaa18-Xaa19-Xaa20-Xaa21-Phe-Xaa23-Xaa24-Trp-L eu-Leu-Xaa28-Xaa29-Xaa30-Xaa31-Ser-Ser-Gly-Gln-Pro-Pro-Pro-Ser-Xaa40 (General Formula 2, SEQ ID NO: 104)
wherein in General Formula 2 above, Xaa1 is 4-imidazoacetyl, histidine, or tyrosine, Xaa2 is glycine, α-methyl-glutamic acid, or Aib, Xaa10 is tyrosine, or cysteine, Xaa13 is alanine, glutamine, tyrosine, or cysteine, Xaa14 is leucine, methionine, or tyrosine, Xaa15 is aspartic acid, glutamic acid, or leucine, Xaa16 is glycine, glutamic acid, or serine, Xaa17 is glutamine, arginine, isoleucine, glutamic acid, cysteine, or lysine, Xaa18 is alanine, glutamine, arginine, or histidine, Xaa19 is alanine, glutamine, cysteine, or valine, Xaa20 is lysine, glutamine, or arginine, Xaa21 is cysteine, glutamic acid, glutamine, leucine, or aspartic acid, Xaa23 is isoleucine or valine, Xaa24 is cysteine, alanine, glutamine, asparagine, or glutamic acid, Xaa28 is lysine, cysteine, asparagine, or aspartic acid, Xaa29 is glycine, glutamine, cysteine, or histidine, Xaa30 is cysteine, glycine, lysine, or histidine, Xaa31 is proline or cysteine, and Xaa40 is cysteine, or is absent.

5. The pharmaceutical composition of claim 1, wherein in General Formula 1 above, Xaa2 is glycine, α-methyl-glutamic acid, or Aib, Xaa7 is threonine, Xaa10 is tyrosine, cysteine, or valine, Xaa12 is lysine or isoleucine, Xaa13 is tyrosine, alanine, or cysteine, Xaa14 is leucine or methionine, Xaa15 is cysteine or aspartic acid, Xaa17 is glutamine, arginine, isoleucine, cysteine, or lysine, Xaa18 is alanine, arginine, or histidine, Xaa19 is alanine, glutamine, or cysteine, Xaa20 is lysine or glutamine, Xaa21 is glutamic acid, cysteine, or aspartic acid, Xaa23 is valine, Xaa24 is alanine, glutamine, cysteine, asparagine, or aspartic acid, and Xaa27 is leucine or lysine.

6. The pharmaceutical composition of claim 2, wherein in General Formula 2 above, Xaa13 is alanine, tyrosine, or cysteine, Xaa15 is aspartic acid or glutamic acid, Xaa17 is glutamine, arginine, cysteine, or lysine, Xaa18 is alanine, arginine, or histidine, Xaa21 is cysteine, glutamic acid, glutamine, or aspartic acid, Xaa23 is isoleucine or valine, Xaa24 is cysteine, glutamine, or asparagine, Xaa28 is cysteine, asparagine, or aspartic acid, Xaa29 is glutamine, cysteine, or histidine, and Xaa30 is cysteine, lysine, or histidine.

7. The pharmaceutical composition of claim 1, wherein in General Formula 1 above, Xaa2 is α-methyl-glutamic acid or Aib, Xaa7 is threonine, Xaa10 is tyrosine or cysteine, Xaa12 is lysine or isoleucine, Xaa13 is tyrosine, alanine, or cysteine, Xaa14 is leucine or methionine, Xaa15 is cysteine or aspartic acid, Xaa16 is glutamic acid, Xaa17 is arginine, isoleucine, cysteine, or lysine, Xaa18 is alanine, arginine, or histidine, Xaa19 is alanine, glutamine, or cysteine, Xaa20 is lysine or glutamine, Xaa21 is glutamic acid or aspartic acid, Xaa23 is valine, Xaa24 is glutamine, asparagine, or aspartic acid, Xaa27 is leucine, and Xaa28 is cysteine, alanine, asparagine, or aspartic acid.

8. The pharmaceutical composition of claim 1, wherein in General Formula 1 above, Xaa1 is histidine or 4-imidazoacetyl, Xaa2 is α-methyl-glutamic acid or Aib, Xaa3 is glutamine, Xaa7 is threonine, Xaa10 is tyrosine, Xaa12 is isoleucine, Xaa13 is alanine or cysteine, Xaa14 is methionine, Xaa15 is aspartic acid, Xaa16 is glutamic acid, Xaa17 is isoleucine or lysine, Xaa18 is alanine or histidine, Xaa19 is glutamine or cysteine, Xaa20 is lysine, Xaa21 is aspartic acid, Xaa23 is valine, Xaa24 is asparagine, Xaa27 is leucine, Xaa28 is alanine or asparagine, Xaa29 is glutamine or threonine, and Xaa30 is cysteine or lysine, or is absent.

9. The pharmaceutical composition of claim 1, wherein the peptide is a peptide comprising an amino acid sequence of General Formula 3 below:
Xaa1-Xaa2-Gln-Gly-Thr-Phe-Thr-Ser-Asp-Tyr-Ser-Lys-Xaa13-Leu-Asp -Glu-Xaa17-Xaa18-Xaa19-Lys-Xaa21-Phe-Val-Xaa24-Trp-Leu-Leu-Xaa28-Xa a29-Xaa30-Xaa31-Ser-Ser-Gly-Gln-Pro-Pro-Pro-Ser-Xaa40 (General Formula 3, SEQ ID NO: 105),
wherein in General Formula 3 above, Xaa1 is histidine or tyrosine, Xaa2 is α-methyl-glutamic acid or Aib, Xaa13 is alanine, tyrosine or cysteine, Xaa17 is arginine, cysteine, or lysine, Xaa18 is alanine or arginine, Xaa19 is alanine or cysteine, Xaa21 is glutamic acid or aspartic acid, Xaa24 is glutamine or asparagine, Xaa28 is cysteine or aspartic acid, Xaa29 is cysteine, histidine, or glutamine, Xaa30 is cysteine or histidine, Xaa31 is proline or cysteine, and Xaa40 is cysteine, or is absent.

10. The pharmaceutical composition of claim 1, wherein R1 is cysteine, GKKNDWKHNIT (SEQ ID NO: 106), CSSGQPPPS (SEQ ID NO: 109), GPSSGAPPPS (SEQ ID NO: 110), GPSSGAPPPSC (SEQ ID NO: 111), PSSGAPPPS (SEQ ID NO: 112), PSSGAPPPSG (SEQ ID NO: 113), PSSGAPPPSHG (SEQ ID NO: 114), PSSGAPPPSS (SEQ ID NO: 115), PSSGQPPPS (SEQ ID NO: 116), or PSSGQPPPSC (SEQ ID NO: 117), or is absent.

11. The pharmaceutical composition of claim 1, wherein the peptide comprises an amino acid sequence selected from the group consisting of SEQ ID NOS: 1 to 102.

12. The pharmaceutical composition of any one of claims 1 to 9, wherein a ring is formed between a 16^{th} amino acid and a 20^{th} amino acid from the N-terminus of the general formulae.

13. The pharmaceutical composition of any one of claims 1 to 11, wherein the C-terminus of the peptide is amidated.

14. The pharmaceutical composition of any one of claims 1 to 11, wherein the pharmaceutical composition has at least one of the effects on increasing LDL absorption, inhibiting the activity of 3-hydroxy-3-methylglutaryl-CoA reductase (HMGCR), and promoting fatty acid degradation.

15. A pharmaceutical composition for preventing or treating hyperlipidemia comprising a conjugate represented by Chemical Formula 1 below,
[Chemical Formula 1] X-Lₐ-F
wherein X is a peptide with activity to a glucagon receptor, a GLP-1 receptor, and a GIP receptor,
L is polyethylene glycol,
a is 0 or a natural number, where when a is 2 or greater, each L is independent,
F is an immunoglobulin Fc region; and
the peptide is a peptide comprising an amino acid sequence represented by General Formula 1 below:
Xaa 1-Xaa2-Xaa3-G ly-Th r-P he-Xaa7-Ser-As p-Xaa 10-Se r-Xaa 12-Xaa 13 -Xaa 14-Xaa 15-Xaa 16-Xaa 17-Xaa 18-Xaa 19-Xaa20-Xaa21 -Phe-Xaa23-Xaa24-Trp-Leu-Xaa27-Xaa28-Xaa29-Xaa30-R1 (General Formula 1, SEQ ID NO: 103)
wherein in General Formula 1 above,
Xaa1 is histidine (His, H), 4-imidazoacetyl (CA), or tyrosine (Tyr, Y),
Xaa2 is glycine (Gly, G), α-methyl-glutamic acid, or aminoisobutyric acid (Aib),
Xaa3 is glutamic acid (Glu, E) or glutamine (Gln, Q),
Xaa7 is threonine (Thr, T) or isoleucine (Ile, I),
Xaa10 is leucine (Leu, L), tyrosine (Tyr, Y), lysine (Lys, K), cysteine (Cys, C), or valine (Val, V),
Xaa12 is lysine (Lys, K), serine (Ser, S), or isoleucine (Ile, I),
Xaa13 is glutamine (Gln, Q), tyrosine (Tyr, Y), alanine (Ala, A), or cysteine (Cys, C),
Xaa14 is leucine (Leu, L), methionine (Met, M), or tyrosine (Tyr, Y),
Xaa15 is cysteine (Cys, C), aspartic acid (Asp, D), glutamic acid (Glu, E), or leucine (Leu, L),
Xaa16 is glycine (Gly, G), glutamic acid (Glu, E), or serine (Ser, S),
Xaa17 is glutamine (Gln, Q), arginine (Arg, R), isoleucine (Ile, I), glutamic acid (Glu, E), cysteine (Cys, C), or lysine (Lys, K),
Xaa18 is alanine (Ala, A), glutamine (Gln, Q), arginine (Arg, R), or histidine (His, H),
Xaa19 is alanine (Ala, A), glutamine (Gln, Q), cysteine (Cys, C), or valine (Val, V),
Xaa20 is lysine (Lys, K), glutamine (Gln, Q), or arginine (Arg, R),
Xaa21 is glutamic acid (Glu, E), glutamine (Gln, Q), leucine (Leu, L), cysteine (Cys, C), or aspartic acid (Asp, D),
Xaa23 is isoleucine (Ile, I) or valine (Val, V),
Xaa24 is alanine (Ala, A), glutamine (Gln, Q), cysteine (Cys, C), asparagine (Asn, N), aspartic acid (Asp, D), or glutamic acid (Glu, E),
Xaa27 is valine (Val, V), leucine (Leu, L), lysine (Lys, K), or methionine (Met, M),
Xaa28 is cysteine (Cys, C), lysine (Lys, K), alanine (Ala, A), asparagine (Asn, N), or aspartic acid (Asp, D),
Xaa29 is cysteine (Cys, C), glycine (Gly, G), glutamine (Gln, Q), threonine (Thr, T), glutamic acid (Glu, E), or histidine (His, H),
Xaa30 is cysteine (Cys, C), glycine (Gly, G), lysine (Lys, K), or histidine (His, H), or is absent, and
R1 is cysteine (Cys, C), GKKNDWKHNIT (SEQ ID NO: 106), m-SSGAPPPS-n (SEQ ID NO: 107), or m-SSGQPPPS-n (SEQ ID NO: 108), or is absent,
wherein m is -Cys-, -Pro-, or -Gly-Pro-, and
n is -Cys-, -Gly-, -Ser-, or -His-Gly-, or is absent.

16. The pharmaceutical composition of claim 15, wherein the immunoglobulin Fc region is an IgG Fc region.

17. The pharmaceutical composition of claim 15, wherein the pharmaceutical composition has at least one of the effects on increasing LDL absorption, inhibiting the activity of 3-hydroxy-3-methylglutaryl-CoA reductase (HMGCR), and promoting fatty acid degradation.

18. The pharmaceutical composition of claim 1, wherein after 24 to 48 hours from administration of the pharmaceutical composition, the activity of 3-hydroxy-3-methylglutaryl-CoA reductase (HMGCR) is less than 50%.

19. The pharmaceutical composition of claim 1, wherein the pharmaceutical composition reduces the triglyceride level and the LDL level in blood.
